# EUROPEAN PATENT APPLICATION

(11) **EP 2 886 664 A1**
(43) Date of publication of application: **24.06.2015**
(21) Application number: 13822588.3
(22) Date of filing: 23.07.2013
(51) Int. Cl.: C12Q 1/68, C12M 1/00, C12N 11/00, C12N 15/115

(54) **MELAMINE ANALYZING NUCLEIC ACID SENSOR, ANALYZING DEVICE, AND ANALYSIS METHOD**

(30) Priority: 27.07.2012 JP 2012167766
(71) Applicant: NEC Solution Innovators, Ltd., Tokyo 136-8627 (JP)
(72) Inventor: HORII Katsunori, Tokyo 136-8627 (JP); KANEKO Naoto, Tokyo 136-8627 (JP); AKITOMI Jou, Tokyo 136-8627 (JP); KATOU Shintarou, Tokyo 136-8627 (JP); FURUICHI Makio, Tokyo 136-8627 (JP); WAGA Iwao, Tokyo 136-8627 (JP)
(74) Representative: Betten & Resch
(86) International application number: PCT/JP2013/069880
(87) International publication number: WO 2014/017471

(57) **Abstract**

The present invention is to provide a new sensor for melamine detection. The nucleic acid sensor for melamine analysis of the present invention includes a polynucleotide (xl) that includes a catalytic nucleic acid molecule (D) that activates a catalytic function and a binding nucleic acid molecule (A) that binds to melamine. The polynucleotide (xl) has any one of the base sequences of SEQ ID NOs: 1 to 14, and n and m are positive integers. In the nucleic acid sensor, since the catalytic function of the catalytic nucleic acid molecule (D) is inhibited in the absence of melamine and the catalytic function of the catalytic nucleic acid molecule (D) is activated in the presence of melamine, melamine can be analyzed by detecting the catalytic function.

SEQUENCE LISTING

<110>  NEC Soft, Ltd

<120>  Nucleic acid sensor, device, and method for analyzing melamin

<130>  1010051024

<150>  JP2012-167766
<151>  2012-07-27

<160>  61

<170>  PatentIn version 3.5

<210>  1
<211>  27
<212>  DNA
<213>  Artificial Sequence

<220>
<223>  nucleic acid sensor


<220>
<221>  misc_feature
<222>  (24)..(24)
<223>  n is one or more Thymine.

<400>  1
gggtgggagg gtcgggccct cgcngcg                                          27


<210>  2
<211>  28
<212>  DNA
<213>  Artificial Sequence

<220>
<223>  nucleic acid sensor


<220>
<221>  misc_feature
<222>  (25)..(25)
<223>  n is one or more Thymine.

<400>  2
gggtgggagg gtcgggccct ccgcngcg                                         28


<210>  3
<211>  29
<212>  DNA
<213>  Artificial Sequence

<220>
<223>  nucleic acid sensor


<220>
<221>  misc_feature
<222>  (26)..(26)

<223>  n is one or more Thymine.

<400>  3
gggtgggagg gtcgggccct ttcgcngcg                                          29


<210>  4
<211>  29
<212>  DNA
<213>  Artificial Sequence

<220>
<223>  nucleic acid sensor


<220>
<221>  misc_feature
<222>  (26)..(26)
<223>  n is one or more Thymine.

<400>  4
gggtgggagg gtcgggcacc ctcgcngcg                                          29


<210>  5
<211>  29
<212>  DNA
<213>  Artificial Sequence

<220>
<223>  nucleic acid sensor


<220>
<221>  misc_feature
<222>  (26)..(26)
<223>  n is one or more Thymine.

<400>  5
gggtgggagg gtcgggccct cccgcngcg                                          29


<210>  6
<211>  30
<212>  DNA
<213>  Artificial Sequence

<220>
<223>  nucleic acid sensor


<220>
<221>  misc_feature
<222>  (26)..(26)
<223>  n is one or more Thymine.

<400>  6
gggtgggagg gtcgggccct cccgcngcgg                                         30


<210>  7
<211>  30
<212>  DNA

<213>  Artificial Sequence

<220>
<223>  nucleic acid sensor


<220>
<221>  misc_feature
<222>  (25)..(25)
<223>  n is one or more Thymine.

<400>  7
gggtgggagg gtcgggcccg cgcgncgcgc                                        30


<210>  8
<211>  31
<212>  DNA
<213>  Artificial Sequence

<220>
<223>  nucleic acid sensor


<220>
<221>  misc_feature
<222>  (26)..(26)
<223>  n is one or more Thymine.

<400>  8
gggtgggagg gtcgggaccc gcgcgncgcg c                                      31


<210>  9
<211>  32
<212>  DNA
<213>  Artificial Sequence

<220>
<223>  nucleic acid sensor


<220>
<221>  misc_feature
<222>  (27)..(27)
<223>  n is one or more Thymine.

<400>  9
gggtgggagg gtcgggcacc cgcgcgncgc gc                                     32


<210>  10
<211>  32
<212>  DNA
<213>  Artificial Sequence

<220>
<223>  nucleic acid sensor


<220>
<221>  misc_feature
<222>  (6)..(6)

<223>  n is one or more Thymine.

<400>  10
gcgcgncgcg ccgcgcgggt gggagggtcg gg                                    32


<210>  11
<211>  33
<212>  DNA
<213>  Artificial Sequence

<220>
<223>  nucleic acid sensor



<220>
<221>  misc_feature
<222>  (6)..(6)
<223>  n is one or more Thymine.

<400>  11
gcgcgncgcg cacgcgcggg tgggagggtc ggg                                   33


<210>  12
<211>  36
<212>  DNA
<213>  Artificial Sequence

<220>
<223>  nucleic acid sensor



<220>
<221>  misc_feature
<222>  (25)..(25)
<223>  n is one or more Thymine.

<220>
<221>  misc_feature
<222>  (29)..(29)
<223>  n is one or more Thymine.

<220>
<221>  misc_feature
<222>  (33)..(33)
<223>  n is one or more Thymine.

<400>  12
gggtgggagg gtcgggccct ccgcnggcng ccngcg                                36


<210>  13
<211>  37
<212>  DNA
<213>  Artificial Sequence

<220>
<223>  nucleic acid sensor



<220>
<221>  misc_feature

<222>  (25)..(25)
<223>  n is one or more Thymine.

<220>
<221>  misc_feature
<222>  (30)..(30)
<223>  n is one or more Thymine.

<220>
<221>  misc_feature
<222>  (34)..(34)
<223>  n is one or more Thymine.

<400>  13
gggtgggagg gtcgggccct ccgcnaggcn gccngcg                                37


<210>  14
<211>  35
<212>  DNA
<213>  Artificial Sequence

<220>
<223>  nucleic acid sensor


<220>
<221>  misc_feature
<222>  (18)..(18)
<223>  n is one or more Thymine.

<400>  14
agggacggga agaacgcngc gaaaatgtgg agggt                                  35


<210>  15
<211>  39
<212>  DNA
<213>  Artificial Sequence

<220>
<223>  nucleic acid sensor

<400>  15
tgggtgggag ggtcgggccc tcgctttttt ttttttgcg                              39


<210>  16
<211>  45
<212>  DNA
<213>  Artificial Sequence

<220>
<223>  nucleic acid sensor

<400>  16
tgggtgggag ggtcgggccc tcgctttttt ttttttgcgt ttttt                       45


<210>  17
<211>  51
<212>  DNA
<213>  Artificial Sequence

<220>
<223>  nucleic acid sensor

<400>  17
tgggtgggag ggtcgggccc tcgctttttt ttttttgcgt tttttttttt t          51


<210>  18
<211>  57
<212>  DNA
<213>  Artificial Sequence

<220>
<223>  nucleic acid sensor

<400>  18
tgggtgggag ggtcgggccc tcgctttttt ttttttgcgt tttttttttt ttttttt          57


<210>  19
<211>  44
<212>  DNA
<213>  Artificial Sequence

<220>
<223>  nucleic acid sensor

<400>  19
tgggtgggag ggtcgggccc tcgctttttt tttttgcgtt tttt          44


<210>  20
<211>  48
<212>  DNA
<213>  Artificial Sequence

<220>
<223>  nucleic acid sensor

<400>  20
gggtgggagg gtcgggaccc gcgcgttttt tttttttcgc gctttttt          48


<210>  21
<211>  43
<212>  DNA
<213>  Artificial Sequence

<220>
<223>  nucleic acid sensor

<400>  21
tgggtgggag ggtcgggccc tcgctttttt ttttgcgttt ttt          43


<210>  22
<211>  49
<212>  DNA
<213>  Artificial Sequence

<220>
<223>  nucleic acid sensor

<400>  22
agcgtgggtg ggagggtcgg gccctcgctt tttttttttt gcgtttttt                    49


<210>  23
<211>  47
<212>  DNA
<213>  Artificial Sequence

<220>
<223>  nucleic acid sensor

<400>  23
   gggtgggagg gtcgggcccg cgcgtttttt ttttttcgcg ctttttt                     47


<210>  24
<211>  49
<212>  DNA
<213>  Artificial Sequence

<220>
<223>  nucleic acid sensor

<400>  24
tttgggtggg agggtcgggc cctttcgctt tttttttttt gcgtttttt                    49


<210>  25
<211>  47
<212>  DNA
<213>  Artificial Sequence

<220>
<223>  nucleic acid sensor

<400>  25
tgggtgggag ggtcgggcac cctcgctttt ttttttttgc gtttttt                      47


<210>  26
<211>  47
<212>  DNA
<213>  Artificial Sequence

<220>
<223>  nucleic acid sensor

<400>  26
   gggtgggagg gtcgggaccc gcgcgttttt ttttttcgcg ctttttt                     47


<210>  27
<211>  49
<212>  DNA
<213>  Artificial Sequence

<220>
<223>  nucleic acid sensor

<400>  27
   gggtgggagg gtcgggcacc cgcgcgtttt ttttttttcg cgctttttt                   49

<210>  28
<211>  44
<212>  DNA
<213>  Artificial Sequence

<220>
<223>  nucleic acid sensor

<400>  28
cacccgcgcg tttttttcgc gcacgcgcgg gtgggagggt cggg                        44


<210>  29
<211>  45
<212>  DNA
<213>  Artificial Sequence

<220>
<223>  nucleic acid sensor

<400>  29
ccacccgcgc gttttttttc gcgccgcgcg ggtgggaggg tcggg                       45


<210>  30
<211>  44
<212>  DNA
<213>  Artificial Sequence

<220>
<223>  nucleic acid sensor

<400>  30
ccacccgcgc gtttttttcg cgccgcgcgg gtgggagggt cggg                        44


<210>  31
<211>  46
<212>  DNA
<213>  Artificial Sequence

<220>
<223>  nucleic acid sensor

<400>  31
ccacccgcgc gttttttttt cgcgccgcgc gggtgggagg gtcggg                      46


<210>  32
<211>  45
<212>  DNA
<213>  Artificial Sequence

<220>
<223>  nucleic acid sensor

<400>  32
cacccgcgcg tttttttttc gcgccgcgcg ggtgggaggg tcggg                        45


<210>  33

<211>  43
<212>  DNA
<213>  Artificial Sequence

<220>
<223>  nucleic acid sensor

<400>  33
cacccgcgcg tttttttcgc gccgcgcggg tgggagggtc ggg                          43


<210>  34
<211>  46
<212>  DNA
<213>  Artificial Sequence

<220>
<223>  nucleic acid sensor

<400>  34
cacccgcgcg tttttttttc gcgcacgcgc gggtgggagg gtcggg                        46


<210>  35
<211>  47
<212>  DNA
<213>  Artificial Sequence

<220>
<223>  nucleic acid sensor

<400>  35
ccacccgcgc gttttttttt cgcgcacgcg cgggtgggag ggtcggg                      47


<210>  36
<211>  46
<212>  DNA
<213>  Artificial Sequence

<220>
<223>  nucleic acid sensor

<400>  36
ccacccgcgc gttttttttc gcgcacgcgc gggtgggagg gtcggg                       46


<210>  37
<211>  46
<212>  DNA
<213>  Artificial Sequence

<220>
<223>  nucleic acid sensor

<400>  37
cacccgcgcg tttttttttt cgcgccgcgc gggtgggagg gtcggg                        46


<210>  38
<211>  40
<212>  DNA
<213>  Artificial Sequence

<220>
<223>  nucleic acid sensor

<400>  38
tgggtgggag ggtcgggccc tccgcttttt tttttttgcg                              40


<210>  39
<211>  40
<212>  DNA
<213>  Artificial Sequence

<220>
<223>  nucleic acid sensor

<400>  39
   tgggtgggag ggtcgggccc tctgcttttt tttttttgcg                              40


<210>  40
<211>  46
<212>  DNA
<213>  Artificial Sequence

<220>
<223>  nucleic acid sensor

<400>  40
agggacggga agaacgcttt tttttttttg cgaaaatgtg gagggt                      46


<210>  41
<211>  39
<212>  DNA
<213>  Artificial Sequence

<220>
<223>  nucleic acid sensor

<400>  41
   gggtgggagg gtcgggccct ccgctttttt ttttttgcg                               39


<210>  42
<211>  41
<212>  DNA
<213>  Artificial Sequence

<220>
<223>  nucleic acid sensor

<400>  42
tgggtgggag ggtcgggccc tcccgctttt ttttttttgc g                           41


<210>  43
<211>  42
<212>  DNA
<213>  Artificial Sequence

<220>
<223>  nucleic acid sensor

<400>  43
tgggtgggag ggtcgggccc tcccgctttt ttttttttgc gg                          42


<210>  44
<211>  52
<212>  DNA
<213>  Artificial Sequence

<220>
<223>  nucleic acid sensor

<400>  44
tgggtgggag ggtcgggccc tccgcttttt tttttttttt tttttttttg cg               52


<210>  45
<211>  64
<212>  DNA
<213>  Artificial Sequence

<220>
<223>  nucleic acid sensor

<400>  45
tgggtgggag ggtcgggccc tccgcttttt tttttttttt tttttttttt tttttttttt      60

tgcg                                                                   64


<210>  46
<211>  76
<212>  DNA
<213>  Artificial Sequence

<220>
<223>  nucleic acid sensor

<400>  46
tgggtgggag ggtcgggccc tccgcttttt tttttttttt tttttttttt tttttttttt      60

tttttttttt tttgcg                                                      76


<210>  47
<211>  58
<212>  DNA
<213>  Artificial Sequence

<220>
<223>  nucleic acid sensor

<400>  47
tgggtgggag ggtcgggccc tccgcttttt aggctttttt ttttttgcct tttttgcg        58


<210>  48
<211>  58
<212>  DNA
<213>  Artificial Sequence

<220>

<223>  nucleic acid sensor

<400>  48
tgggtgggag ggtcgggccc tccgcttttt tggctttttt ttttttgcct tttttgcg          58


<210>  49
<211>  64
<212>  DNA
<213>  Artificial Sequence

<220>
<223>  nucleic acid sensor

<400>  49
agggacggga agaacgcttt tttttttttt tttttttttt tttttttgcg aaaatgtgga          60

gggt                                                                        64


<210>  50
<211>  70
<212>  DNA
<213>  Artificial Sequence

<220>
<223>  nucleic acid sensor

<400>  50
agggacggga agaacgcttt tttttttttt tttttttttt tttttttttt tttgcgaaaa          60

tgtggagggt                                                                  70


<210>  51
<211>  76
<212>  DNA
<213>  Artificial Sequence

<220>
<223>  nucleic acid sensor

<400>  51
agggacggga agaacgcttt tttttttttt tttttttttt tttttttttt tttttttttg          60

cgaaaatgtg gagggt                                                           76


<210>  52
<211>  60
<212>  DNA
<213>  Artificial Sequence

<220>
<223>  nucleic acid sensor

<400>  52
tgggtgggag ggtcgggccc tcccgctttt tttttttttt tttttttttt ttttttgcgg          60


<210>  53
<211>  66
<212>  DNA

<213>  Artificial Sequence

<220>
<223>  nucleic acid sensor

<400>  53
tgggtgggag ggtcgggccc tcccgctttt tttttttttt tttttttttt tttttttttt      60

ttgcgg                                                                  66


<210>  54
<211>  72
<212>  DNA
<213>  Artificial Sequence

<220>
<223>  nucleic acid sensor

<400>  54
tgggtgggag ggtcgggccc tcccgctttt tttttttttt tttttttttt tttttttttt      60

ttttttttgc gg                                                           72


<210>  55
<211>  58
<212>  DNA
<213>  Artificial Sequence

<220>
<223>  nucleic acid sensor

<400>  55
tgggtgggag ggtcgggccc tccgcttttt tttttttttt tttttttttt tttttgcg        58


<210>  56
<211>  70
<212>  DNA
<213>  Artificial Sequence

<220>
<223>  nucleic acid sensor

<400>  56
tgggtgggag ggtcgggccc tccgcttttt tttttttttt tttttttttt tttttttttt      60

tttttttgcg                                                              70


<210>  57
<211>  10
<212>  DNA
<213>  Artificial Sequence

<220>
<223>  nucleic acid sensor


<220>
<221>  misc_feature
<222>  (4)..(4)

<223>  n is one or more Thymine.

<400>  57
cgcngcg                                                                 7


<210>  58
<211>  12
<212>  DNA
<213>  Artificial Sequence

<220>
<223>  nucleic acid sensor


<220>
<221>  misc_feature
<222>  (6)..(6)
<223>  n is one or more Thymine.

<400>  58
gcgcgncgcg cg                                                          12


<210>  59
<211>  14
<212>  DNA
<213>  Artificial Sequence

<220>
<223>  nucleic acid sensor

<400>  59
agggacggga agaa                                                        14


<210>  60
<211>  14
<212>  DNA
<213>  Artificial Sequence

<220>
<223>  nucleic acid sensor

<400>  60
aaaatgtgga gggt                                                        14


<210>  61
<211>  16
<212>  DNA
<213>  Artificial Sequence

<220>
<223>  nucleic acid sensor

<400>  61
gggtgggagg gtcggg                                                      16

## Description

### Technical Field

The present invention relates to a nucleic acid sensor for melamine analysis, a device for melamine analysis, and a method for melamine analysis.

### Background Art

Melamine is an organic nitrogen compound with a high content of nitrogen. In recent years, there is a problem that melamine is mixed in a processed product such as milk, milk powder, or the like for increasing an apparent protein amount and this results in a high incidence of renal failure in infants. For preventing the ingestion and distribution of such melamine-containing food, great importance is placed on the analysis of melamine in food. While the guideline value of a melamine concentration according to the Japanese Ministry of Health is 0.5 ppm, virtually, it is acceptable if the melamine concentration of about 500 ppm can be detected in terms of effects on human body and the like.

Currently, the Fourier Transform Infrared Spectroscopy (FTIR) is used for the analysis of melamine. However, since an analyzer for FTIR is an extremely expensive large apparatus, for example, there is a problem that it is not easy for importers, distributors, retailers, and consumers of food to use the analyzer and they have no choice but to ask an inspection agency that owns the analyzer to conduct the analysis.

Hence, as a new detection method, a method using a nucleic acid molecule (so-called aptamer) that binds to melamine is being developed (Non-Patent Document 1). Specifically, a method of analyzing a catalytic resonance scattering spectrum using a colloidal gold nanoparticle on which the nucleic acid molecule is immobilized is being proposed. According to this method, the catalytic ability of the colloidal particle is deactivated when melamine is bound between the nucleic acid molecules immobilized on the colloidal particle. Thus, it is considered that the presence or absence and the amount of melamine in a sample can be analyzed by analyzing the presence or absence and the decrease in the catalytic ability by the catalytic resonant scattering spectrum. However, this method also requires a special apparatus and is far from an easy analysis.

### Citation List

### Non-Patent Document(s)

Non-Patent Document 1: A Highly Sensitive Aptamer-Nanogold Catalytic Resonance Scattering Spectral Assay for Melamine. Aihui Liang, J Fluoresc (2011) 21:1907-1912

### Summary of Invention

### Problem to be Solved by the Invention

Hence, the present invention is intended to provide a new sensor for melamine analysis.

### Means for Solving Problem

The present invention provides a nucleic acid sensor for melamine analysis including: the following polynucleotide (x1), (x2), (x3), or (x4) that includes a catalytic nucleic acid molecule (D) that activates a catalytic function and a binding nucleic acid molecule (A) that binds to melamine:
(x1) a polynucleotide having any one of base sequences of SEQ ID NOs: 1 to 14, wherein n and m are positive integers;
(x2) a polynucleotide having a base sequence obtained by substitution, deletion, addition, and/or
insertion of one or more bases in the base sequence of the polynucleotide (x1), wherein a catalytic function of the catalytic nucleic acid molecule (D) is inhibited in the absence of melamine and the catalytic function of the catalytic nucleic acid molecule (D) is activated in the presence of melamine;
(x3) a polynucleotide having a base sequence having at least 80% identity to any base sequence of the polynucleotide (x1), wherein the catalytic function of the catalytic nucleic acid molecule (D) is inhibited in the absence of melamine and the catalytic function of the catalytic nucleic acid molecule (D) is activated in the presence of melamine; and
(x4) a polynucleotide having a base sequence complementary to a polynucleotide that hybridizes to a polynucleotide having any base sequence of the polynucleotide (x1) under a stringent condition, wherein the catalytic function of the catalytic nucleic acid molecule (D) is inhibited in the absence of melamine and the catalytic function of the catalytic nucleic acid molecule (D) is activated in the presence of melamine.

The present invention also provides a device for melamine analysis including: a base material; a nucleic acid sensor; and a detection unit, wherein the nucleic acid sensor and the detection unit are arranged on the base material, the nucleic acid sensor is the nucleic acid sensor according to the present invention, and the detection unit is a detection unit detecting the catalytic function of the catalytic nucleic acid molecule (D) in the nucleic acid sensor.

The present invention also provides a kit for analysis including: a container; and a nucleic acid sensor, wherein the nucleic acid sensor is the nucleic acid sensor according to the present invention.

The present invention also provides a method for melamine analysis including: a contact step of bringing a sample into contact with the nucleic acid sensor for melamine analysis according to the present invention; and a detection step of detecting the catalytic function of the catalytic nucleic acid molecule (D) in the nucleic acid sensor to detect melamine in the sample.

The present invention also provides a method for melamine analysis including: a contact step of bringing a sample into contact with the device for analysis according to the present invention; and a detection step of detecting the catalytic function of the catalytic nucleic acid molecule (D) of the nucleic acid sensor in the detection unit of the device for analysis to detect melamine in the sample.

### Effects of the Invention

According to the nucleic acid sensor of the present invention, it is possible to switch ON-OFF of the catalytic function of the catalytic nucleic acid molecule (D) depending on whether or not the binding nucleic acid molecule (A) binds to melamine. Therefore, by detecting the catalytic function of the catalytic nucleic acid molecule (D), the presence or absence or the amount of melamine can be detected without difficulty. Furthermore, since the analysis device of the present invention uses the nucleic acid sensor as described above, for example, the reduction of the size of the device and the chipping of the device can be achieved and a simple analysis can be achieved even with respect to a number of specimens. Therefore, the present invention can be a very useful technique in the melamine analysis, for example. In the present invention, "analysis" is a concept including, for example, a quantitative analysis, a semi-quantitative analysis, and qualitative analysis.

### Brief Description of Drawings

[FIG. 1] FIG. 1 shows schematic views of the structures of nucleic acid sensors in Example 1 of the present invention.
[FIG. 2] FIG. 2 is a graph showing the results of absorbance measurement in Example 1 of the present invention.
[FIG. 3] FIG. 3 shows schematic views of the structures of nucleic acid sensors in Example 2 of the present invention.
[FIG. 4] FIG. 4 is a graph showing the results of absorbance measurement in Example 2 of the present invention.
[FIG. 5] FIG. 5 is a graph showing the results of RLU measurement in Example 3 of the present invention.
[FIG. 6] FIG. 6 shows schematic views of the structures of nucleic acid sensors in Example 4 of the present invention.
[FIG. 7] FIG. 7 is a graph showing the results of RLU measurement in Example 4 of the present invention.
[FIG. 8] FIG. 8 shows schematic views of the structures of nucleic acid sensors in Example 5 of the present invention.
[FIG. 9] FIG. 9 is a graph showing the results of RLU measurement in Example 5 of the present invention.
[FIG. 10] FIG. 10 shows schematic views of the structures of nucleic acid sensors in Example 6 of the present invention.
[FIG. 11] FIG. 11 is a graph showing the results of RLU measurement in Example 6 of the present invention.
[FIG. 12] FIG. 12 shows schematic views of the structures of nucleic acid sensors in Example 7 of the present invention.
[FIG. 13] FIG. 13 is a graph showing the results of RLU measurement in Example 7 of the present invention.
[FIG. 14] FIG. 14 shows schematic views of the structures of nucleic acid sensors in Example 8 of the present invention.
[FIG. 15] FIG. 15 is a graph showing the results of RLU measurement in Example 8 of the present invention.
[FIG. 16] FIG. 16 is a photograph showing the results of colorimetry in Example 9 of the present invention.

### Description of Exemplary Embodiment

### (Nucleic acid sensor and analysis method using the same)

As described above, the nucleic acid sensor for melamine analysis according to the present invention includes the following polynucleotide (x1), (x2), (x3), or (x4) that includes a catalytic nucleic acid molecule (D) that activates a catalytic function and a binding nucleic acid molecule (A) that binds to melamine:
(x1) a polynucleotide having any one of base sequences of SEQ ID NOs: 1 to 14, wherein n and m are positive integers;
(x2) a polynucleotide having a base sequence obtained by substitution, deletion, addition, and/or insertion of one or more bases in the base sequence of the polynucleotide (x1), wherein a catalytic function of the catalytic nucleic acid molecule (D) is inhibited in the absence of melamine and the catalytic function of the catalytic nucleic acid molecule (D) is activated in the presence of melamine;
(x3) a polynucleotide having a base sequence having at least 80% identity to any base sequence of the polynucleotide (x1), wherein the catalytic function of the catalytic nucleic acid molecule (D) is inhibited in the absence of melamine and the catalytic function of the catalytic nucleic acid molecule (D) is activated in the presence of melamine; and
(x4) a polynucleotide having a base sequence complementary to a polynucleotide that hybridizes to a polynucleotide having any base sequence of the polynucleotide (x1) under a stringent condition, wherein the catalytic function of the catalytic nucleic acid molecule (D) is inhibited in the absence of melamine and the catalytic function of the catalytic nucleic acid molecule (D) is activated in the presence of melamine.

SEQ ID NO: 1 GGGTGGGAGGGTCGGGccctCGC(T)ₙGCG
SEQ ID NO: 2 GGGTGGGAGGGTCGGGccctcCGC(T)ₙGCG
SEQ ID NO: 3 GGGTGGGAGGGTCGGGccctttCGC(T)ₙGCG
SEQ ID NO: 4 GGGTGGGAGGGTCGGGcaccctCGC(T)ₙGCG
SEQ ID NO: 5 GGGTGGGAGGGTCGGGccctccCGC(T)ₙGCG
SEQ ID NO: 6 GGGTGGGAGGGTCGGGccctccCGC(T)ₙGCGg
SEQ ID NO: 7 GGGTGGGAGGGTCGGGcccGCGCG(T)ₙCGCGC
SEQ ID NO: 8 GGGTGGGAGGGTCGGGacccGCGCG(T)ₙCGCGC
SEQ ID NO: 9 GGGTGGGAGGGTCGGGcacccGCGCG(T)ₙCGCGC
SEQ ID NO: 10 GCGCG(T)ₙCGCGCcgcgcGGGTGGGAGGGTCGGG
SEQ ID NO: 11 GCGCG(T)ₙCGCGCacgcgcGGGTGGGAGGGTCGGG
SEQ ID NO: 12 GGGTGGGAGGGTCGGGccctcCGC(T)ₘGGC(T)ₙGCC(T)ₘGCG
SEQ ID NO: 13 GGGTGGGAGGGTCGGGccctcCGC(T)ₘAGGC(T)ₙGCC(T)ₘGCG
SEQ ID NO: 14 AGGGACGGGAAGAACGC(T)ₙGCGAAAATGTGGAGGGT

In the base sequences of SEQ ID NOs: 1 to 13, the underlined parts indicate the binding nucleic acid molecules (A) and the catalytic nucleic acid molecules (D). Specifically, in the base sequences of SEQ ID NOs: 1 to 9, 12, and 13, for example, the underlined parts on the 5' side indicate the catalytic nucleic acid molecules (D) and the underlined parts on the 3' side indicate the binding nucleic acid molecules (A). In SEQ ID NOs: 10 and 11, for example, the underlined parts on the 5' side indicate the binding nucleic acid molecules (A) and the underlined parts on the 3' side indicate the catalytic nucleic acid molecules (D). In the base sequence of SEQ ID NO: 14, the underlined part indicates the binding nucleic acid molecule (A) and the sequences on the both sides of the underlined part indicate double-stranded catalytic nucleic acid molecules (D).

The nucleic acid sensor of the present invention may further include, for example, a linker sequence (L) that links the binding nucleic acid molecule (A) and the catalytic nucleic acid molecule (D). The linker sequence (L) has a length of, for example, 0 to 14 bases, preferably 0 to 10 bases, and more preferably 0 to 7 bases. In each of the base sequences of SEQ ID NOs: 1 to 13, for example, a sequence between the underlined regions is the linker sequence (L), and each of the base sequences of SEQ ID NOs: 1 to 13 may have a further linker sequence (L).

The binding nucleic acid molecule (A) can be any nucleic acid molecule as long as it binds to melamine. Preferably, the binding nucleic acid molecule (A) binds to melamine in its molecule. Hereinafter, the binding nucleic acid molecule (A) is also referred to as a melamine aptamer. The binding nucleic acid molecule (A) is formed of a stem-forming region S_{A}, a loop region, and a stem-forming region S_{A'}, for example. The stem-forming region S_{A} and the stem-forming region S_{A'} have the sequences complementary to each other, and, for example, form a stem by self annealing in the presence of melamine.

The stem-forming region S_{A} and the stem-forming region S_{A'} each have a length of, for example, 1 to 10 bases, preferably 2 to 7 bases, and more preferably 3 to 5 bases. The length of the stem-forming region S_{A} may be the same as or different from the length of the stem-forming region S_{A'}, and the former case is preferable. More preferably, these regions have the sequences perfectly complementary to each other.

The loop region is, for example, a polynucleotide of deoxythymidine triphosphate (dTTP) (hereinafter, poly dT), and can be represented by (T)ₙ. n is a positive integer. n is, for example, 3 to 100, preferably 6 to 70, more preferably 6 to 60 or 10 to 60, and yet more preferably 6 to 48 or 12 to 48.

The binding nucleic acid molecule (A) may also have an internal loop region having poly dT in addition to a loop region having poly dT, for example. The binding nucleic acid molecule (A) of this type is also called, for example, a tandem type.

The length of the binding nucleic acid molecule (A) is not particularly limited, and the length is, for example, 9 to 120 bases, preferably 20 to 70 bases, and more preferably 18 to 54 bases.

Specific examples of the sequences of the binding nucleic acid molecule (A) are as follows:
CGC(T)ₙGCG (SEQ ID NO: 57)
   Me101 n = 7
   Me102 n = 8
   Me103 n = 9
   Me104 n = 10
   Me105 n= 11
   Me106 n = 12
GCGCG(T)ₙCGCGCG (SEQ ID NO: 58)
   Me107 n = 7
   Mel08 n = 8
   Me109 n = 9
   Me110 n = 10
   Me111 n= 11
   Me112 n = 12

The catalytic nucleic acid molecule (D) can be any nucleic acid molecule as long as it activates a catalytic function. The catalytic function is, for example, the catalytic function of a redox reaction. The redox reaction can be any reaction, for example, as long as it causes electron transfer between two substrates in the process of producing a product from a substrate. The type of the redox reaction is not particularly limited. Examples of the catalytic function of a redox reaction include the activities similar to those of enzyme, and specific examples thereof include the activities similar to those of peroxidase (hereinafter, referred to as "peroxidase-like activity"). An example of the peroxidase activity includes the horseradish peroxidase (HRP) activity. The catalytic nucleic acid molecule (D) can be called DNA enzyme or DNAzyme in the case of DNA that will be described below, and can be called RNA enzyme or RNAzyme in the case of RNA that will be described below.

The catalytic nucleic acid molecule (D) is preferably a nucleic acid that forms the structure of G-quartet (or is also called G-tetrad) and is more preferably a nucleic acid that forms the structure of guanine quadruplex (or is also called G-quadruplex). The G-tetrad is, for example, a planar structure formed of four guanine bases, and G-quadruplex is, for example, the structure in which plural G-tetrads are stacked on top of each other. The G-tetrad and the G-quadruplex are formed in the nucleic acid that repeatedly includes the structural motif of G-rich, for example. The G-tetrad includes, for example, a parallel type and an antiparallel type, and the parallel type is preferable.

The catalytic nucleic acid molecule (D) is preferably a nucleic acid that is bindable to porphyrin. Specifically, the catalytic nucleic acid molecule (D) is preferably a nucleic acid that forms a G-tetrad and is bindable to porphyrin. The nucleic acid that forms a G-tetrad is known to activate the above-described catalytic function of the redox reaction by binding to porphyrin to form a complex, for example. In the nucleic acid sensor, it is preferable that the catalytic nucleic acid molecule (D) is inhibited from binding to porphyrin when a stem is formed between the catalytic nucleic acid molecule (D) and the linker sequence (L) or the binding nucleic acid molecule (A) in the state where melamine is not bound to the binding nucleic acid molecule (A), and the catalytic nucleic acid molecule (D) is allowed to bind to porphyrin when melamine binds to the binding nucleic acid molecule (A) and thus the formation of the stem is released, for example. Specifically, in the nucleic acid sensor, it is preferable that the catalytic nucleic acid molecule (D) is inhibited from forming the G-tetrad and thus inhibited from binding to porphyrin in the state where melamine is not bound to the binding nucleic acid molecule (A), and the catalytic nucleic acid molecule (D) is allowed to form the G-tetrad and to bind to porphyrin when melamine binds to the binding nucleic acid molecule (A), for example.

The porphyrin is not particularly limited, and examples thereof include unsubstituted porphyrins and the derivatives thereof. Examples of the derivatives include substituted porphyrins and metal porphyrins obtained by forming complexes with metal elements. An example of the substituted porphyrin includes N-Methylmesoporphyrin. An example of the metal porphyrin includes hemin, which is a ferric complex. For example, the porphyrin is preferably the metal porphyrin and is more preferably hemin.

The catalytic nucleic acid molecule (D) may be, for example, a single-stranded nucleic acid molecule or a double-stranded nucleic acid molecule. In the nucleic acid sensor, in the case where the catalytic nucleic acid molecule (D) is a single-stranded nucleic acid molecule, for example, the catalytic nucleic acid molecule (D) is inhibited from forming the G-tetrad when a stem is formed between the catalytic nucleic acid molecule (D) and the linker sequence (L) or the binding nucleic acid molecule (A) in the state where melamine is not bound to the binding nucleic acid molecule (A), and the catalytic nucleic acid molecule (D) is allowed to form the G-tetrad when melamine binds to the binding nucleic acid molecule (A) and thus the formation of the stem is released. Furthermore, in the nucleic acid sensor, in the case where the catalytic nucleic acid molecule (D) is a double-stranded nucleic acid molecule, for example, the catalytic nucleic acid molecule (D) is inhibited from forming the G-tetrad in the state where melamine is not bound to the binding nucleic acid molecule (A) because the structure of the binding nucleic acid molecule (A) vibrates, and the catalytic nucleic acid molecule (D) is allowed to form the G-tetrad when melamine binds to the binding nucleic acid molecule (A). In the case where the catalytic nucleic acid molecule (D) is a single-stranded nucleic acid molecule, the length is, for example, 15 to 30 bases, preferably 15 to 24 bases, and more preferably 15 to 18 bases. In the case where the catalytic nucleic acid molecule (D) is a double-stranded nucleic acid molecule, the length of each of the strands is, for example, 7 to 21 bases, preferably 7 to 17 bases, and more preferably 7 to 14 bases.

An example of the double-stranded catalytic nucleic acid molecule (D) includes the combination of the following sequences.

| | |
|---|---|
| 5' -AGGGACGGGAAGAA-3' | (SEQ ID NO: 59) |
| 3'-TGGGAGGTGTAAAA-5' | (SEQ ID NO: 60) |

An example of the single-stranded catalytic nucleic acid molecule (D) includes the following sequence.
neco0584 (SEQ ID NO: 61)
   5'-GGGTGGGAGGGTCGGG-3'

As described above, it is preferable that the catalytic nucleic acid molecule (D) is inhibited from activating a catalytic function when a stem is formed between the catalytic nucleic acid molecule (D) and the linker sequence (L) in the state where melamine is not bound to the binding nucleic acid molecule (A) in the absence of melamine, and the catalytic nucleic acid molecule (D) is allowed to activate the catalytic function when melamine binds to the binding nucleic acid molecule (A) and thus the formation of the stem is released, for example. Therefore, as described above, preferably, the nucleic acid sensor further includes a linker sequence (L) between the catalytic nucleic acid molecule (D) and the binding nucleic acid molecule (A) to link them. The base sequence of the linker sequence (L) can be set according to the sequence of the catalytic nucleic acid molecule, for example.

As described above, the nucleic acid sensor according to the present invention includes the polynucleotide (x1), (x2), (x3), or (x4) that includes the catalytic nucleic acid molecule (D) and the binding nucleic acid molecule (A). The polynucleotide (x1), (x2), (x3), or (x4) is a polynucleotide in which the catalytic function of the catalytic nucleic acid molecule (D) is inhibited in the absence of melamine and the catalytic function of the catalytic nucleic acid molecule (D) is activated in the presence of melamine.

The polynucleotide (x1) has any one of the base sequences of SEQ ID NOs: 1 to 14, and n and m are positive integers. Examples of n include the aforementioned numerical values and n is preferably 6 to 48. In the nucleotides of SEQ ID NOs: 12 and 13, since two (T)ₘs form an internal loop, each m is, for example, 2 to 31, preferably 2 to 24, more preferably 3 to 17, and yet more preferably 4 to 10.

The polynucleotide (x2) has a base sequence obtained by substitution, deletion, addition, and/or insertion of one or more bases in the base sequence of the polynucleotide (x1). In the polynucleotide (x2), there is no limitation on "one or more". The number of the substituted bases in the base sequence of the polynucleotide (x1) is, for example, 1 to 5, preferably 1 to 4, more preferably 1 to 3, yet more preferably 1 or 2, and particularly preferably 1. The number of the added or inserted bases in the base sequence of the polynucleotide (x1) is, for example, 1 to 5, preferably 1 to 4, more preferably 1 to 3, yet more preferably 1 or 2, and particularly preferably 1. The number of the deleted bases in the base sequence of the polynucleotide (x1) is, for example, 1 to 5, preferably 1 to 4, more preferably 1 to 3, yet more preferably 2 or 1, and particularly preferably 1.

The polynucleotide (x3) has a base sequence having at least 80% identity to any base sequence of the polynucleotide (x1). In the polynucleotide (x3), the identity to the base sequence of the polynucleotide (x1) is, for example, at least 70%, preferably at least 80% or at least 85%, yet more preferably at least 90%, still more preferably at least 95%, at least 96%, at least 97%, or at least 98%, and particularly preferably at least 99%. The identity can be calculated using BLAST or the like under a default condition, for example.

The polynucleotide (x4) has a base sequence complementary to a polynucleotide that hybridizes to a polynucleotide having any base sequence of the polynucleotide (x1) under a stringent condition. In the polynucleotide (x4), "hybridizes under a stringent condition" refers to an experimental condition of hybridization well known to those skilled in the technical field, for example. Specifically, "stringent condition" refers to a condition in which a base sequence can be identified by hybridizing at 60 to 68°C in the presence of 0.7 to 1 mol/L NaCl and then washing at 65 to 68°C using 0.1 to 2 × SSC solution, for example. 1 × SSC contains 150 mmol/L NaCl and 15 mmol/L sodium citrate.

The nucleic acid sensor may be, for example, a sensor having any of the polynucleotides (x1) to (x4) or a sensor including any of the polynucleotides (x1) to (x4). In the latter case, for example, the sensor may further include either a 5' end-additional sequence linked to the 5' end of the polynucleotide or a 3' end-additional sequence linked to the 3' end of the polynucleotide or include both of the 5' end-additional sequence and the 3' end-additional sequence. The 5' end-additional sequence has a length of, for example, 0 to 22 bases, preferably 0 to 18 bases, and more preferably 0 to 14 bases, and the 3' end-additional sequence has a length of, for example, 0 to 18 bases, preferably 0 to 10 bases, and more preferably 0 to 7 bases. As will be described below, when the nucleic acid sensor is used in the immobilized state, for example, the nucleic acid sensor may further include the 5' end-additional sequence or the 3' end-additional sequence as a linker sequence for immobilization. An example of the linker sequence for immobilization includes poly dT. The length of the linker sequence for immobilization is not particularly limited, and the length is, for example, 0 to 120 bases and preferably 6 to 80 bases.

Specific examples of the nucleic acid sensor include sensors respectively including polynucleotides (X1), (X2), (X3), and (X4). In these polynucleotides, for example, the catalytic function of the catalytic nucleic acid molecule (D) is inhibited in the absence of melamine and the catalytic function of the catalytic nucleic acid molecule (D) is activated in the presence of melamine.

The polynucleotide (X1) includes the base sequence of the polynucleotide (x1) and has any one of the base sequences of SEQ ID NOs: 15 to 56. These sequences are shown in Tables 1 to 4.

**[Table 1]**

| Sensor No. | name | sequence | SEQ ID NO |
|---|---|---|---|
| 0 | N1Mel06_I1_A0_D3_T00 | tGGGTGGGAGGGTCGGGccctCGTTTTTTTTTTTTGCG | 15 |
| 22 | N1Mel06_I1_A0_D3_06 | tGGGTGGGAGGGTCGGGccctCGTTTTTTTTTTTTGCGtttttt | 16 |
| 23 | N1Mel06_I1_A0_D3_T12 | tGGGTGGGAGGGTCGGGccctCGTTTTTTTTTTTTGCGtttttttttttt | 17 |
| 24 | N1Mel06_I1_A0_D3_T18 | tGGGTGGGAGGGTCGGGccctCGTTTTTTTTTTTTGCGtttttttttttttttttt | 18 |
| 25 | N1Mel05_I1_A0_D3_T06 | tGGGTGGGAGGGTCGGGccctCGTTTTTTTTTTTGCGtttttt | 19 |
| 27 | N1Mel04_I1_A0_D3_T06 | tGGGTGGGAGGGTCGGGccctCGTTTTTTTTTTTTGCGtttttt | 21 |
| 28 | N1Mel06_I1_A4_D3_T06 | agcgtGGGTGGGAGGGTCGGGccctCGCTTTTTTTTTTTTGCGtttttt | 22 |
| 1 | N1Mel06_I1_A0_D3_T00_C | TGGGTGGGAGGGTCGGGCCCTCCGCTTTTTTTTTTTTGCG | 38 |
| | N1Mel06_I1_AD_D3_T00_T | TGGGTGGGAGGGTCGGGCCCTCTGCTTTTTTTTTTTTTGCG | 39 |
| 3 | N1Mel06_I1_A0_D3C_1 | GGGTGGGAGGGTCGGGCCCTCCGGTTTTTTTTTTTTTGCG | 41 |
| 6 | N1Mel06_I1_A0_D3C_H24 | TGGGTGGGAGGGTCGGGCCCTCCGCTTTTTTTTTTTTTTTTTTTTTTTTGCG | 44 |
| 7 | N1Mel06_I1_A0_D3C_H36 | TGGGTGGGAGGGTCGGGCCCTCCGCTTTTTTTTTTTTTTTTTTTTTTTTTTTTTTTTTTTTGCG | 45 |
| 8 | N1Mel06_I1_A0_D3C_H48 | TGGGTGGGAGGGTCGGGCCCTCCGCTTTTTTTTTTTTTTTTTTTTTTTTTTTTTTTTTTTTTTTTTTTTTTTTGCG | 46 |
| 11 | N1Mel06_I1_A0_D3C_H30 | TGGGTGGGAGGGTCGGGCCCTCCGCTTTTTTTTTTTTTTTTTTTTTTTTTTTTTGCG | 55 |
| 12 | N1Mel06_I1_A0_D3C_H42 | TGGGTGGGAGGGTCGGGCCCTCCGCTTTTTTTTTTTTTTTTTTTTTTTTTTTTTTTTTTTTTTTTTTGCG | 56 |

Each of the polynucleotides (X1) shown in Table 1 includes the base sequence of SEQ ID NO: 1 or 2 and includes an additional sequence of 0 to 5 bases (0, 1, or 5 bases) at the 5' end of the base sequence of SEQ ID NO: 1 or 2 and an additional sequence of 0 to 12 bases (0, 6, 12, or 16 bases) at the 3' end of the base sequence of SEQ ID NO: 1 or 2.

**[Table 2]**

| Sensor No. | name | sequence | SEQ ID NO |
|---|---|---|---|
| 30 | N1Mel06_I3_A0_D3_T06 | tttGGGTGGGtGGGTAGGGTCGGGccctttCGCTTTTTTTTTTTTGCGtttttt | 24 |
| 31 | N1Mel06_I1_A0_D5_T06 | tGGGTGGGAGGGTCGGGcaccctCGTTTTTTTTTTTTGCGtttttt | 25 |
| 4 | N1Mel06_I1_A0_D3C_C | TGGGTGGGAGGGTCGGGCCCTCCGCTTTTTTTTTTTTGCG | 42 |
| 5 | N1Mel06_I1_A0_D3C_CG | TGGGTGGGAGGGTCGGGCCCTCCGCTTTTTTTTTTTTGCGG | 43 |
| 16 | N1Mel06_I1_A0_D3C_CG_H30 | TGGGTGGGAGGGTCGGGCCCTCCGCTTTTTTTTTTTTTTTTTTTTTTTTTTTTTTGCGG | 52 |
| 17 | N1Mel06_I1_A0_D3C_CG_H36 | TGGGTGGGAGGGTCGGGCCCTCCGCTTTTTTTTTTTTTTTTTTTTTTTTTTTTTTTTTTTTGCGG | 53 |
| 18 | N1Mel06_I1_A0_D3C_CG_H42 | TGGGTGGGAGGGTCGGGCCCTCCGCTTTTTTTTTTTTTTTTTTTTTTTTTTTTTTTTTTTTTTTTTTGCGG | 54 |
| 29 | N1Mel12_I0_A0_D3_T06 | GGGTGGGAGGGTCGGGcccGCGCGTTTTTTTTTTTTCGCGCtttttt | 23 |
| 26 | N1Mel12_I0_A0_D4_T06 | GGGTGGGAGGGTCGGGacccGCGCGTTTTTTTTTTTTTCGCGCtttttt | 20 |
| 32 | N1Mel11_I0_A0_D4_T06 | GGGTGGGAGGGTCGGGacccGCGCGTTTTTTTTTTTTCGCGCtttttt | 26 |
| 33 | N1Mel12_I0_A0_D5_T06 | GGGTGGGAGGGTCGGGacccGCGCGTTTTTTTTTTTTCGCGCtttttt | 27 |

Each of the polynucleotides (X1) shown in Table 2 includes the base sequence of SEQ ID NO: 3, 4, 5, 7, 8, or 9 and includes an additional sequence of 0 to 3 bases (0, 1, or 3 bases) at the 5' end of the base sequence of SEQ ID NO: 3, 4, 5, 7, 8, or 9 and an additional sequence of 0 to 6 bases (0 or 6 bases) at the 3' end of the base sequence of SEQ ID NO: 3, 4, 5, 7, 8, or 9.

**[Table 3]**

| name | sequence | SEQ ID NO |
|---|---|---|
| Mel07_N1_S0_I0_A6_D5_T00 | CACCCGCGCGTTTTTTTCGCGCACGCGCGGGTGGGAGGGTCGGG | 28 |
| Mel08_N1_S0_I0_A5_D6_T00 | CCACCCGCGCGTTTTTTTTTCGCGCGCGCGGGTGGGAGGGTCGGG | 29 |
| Mel07_N1_S0_I0_A5_D6_T00 | CCACCCGCGCGTTTTTTTCGCGCCGCGCGGGTGGGAGGGTCGGG | 30 |
| Mel08_N1_S0_I0_A5_D6_T00 | CCACCCGCGCGTTTTTTTTTCGCGCCGCGCGGGTGGGAGGGTCGGG | 31 |
| Mel09_N1_S0_I0_A5_D5_T00 | CACCCGCGCGTTTTTTTTTCGCGCCGCGCGGGTGGGAGGGTCGGG | 32 |
| Mel07_N1_S0_I0_A5_D5_T00 | CACCCGCGCGTTTTTTTCGCGCCGCGCGGGTGGGAGGGTCGGG | 33 |
| Mel09_N1_S0_I0_A6_D5_T00 | CACCCGCGCGTTTTTTTTTCGCGCACGCGCGGGTGGGAGGGTCGGG | 34 |
| Mel03_N1_S0_I0_A6_D6_T00 | CCACCCGCGCGTTTTTTTTTCGCGCACGCGCGGGTGGGAGGGTCGGG | 35 |
| Mel08_N1_S0_I0_A6_D6_T00 | CCACCCGCGCGTTTTTTTTCGCGCACGCGCGGGTGGGAGGGTCGGG | 36 |
| Mel10_N1_S0_I0_A5_D5_T00 | CACCCGCGCGTTTTTTTTTTCGCGCCGCGCGGGTGGGAGGGTCGGG | 37 |

Each of the polynucleotides (X1) shown in Table 3 includes the base sequence of SEQ ID NO: 10 or 11 and includes an additional sequence of 5 or 6 bases at the 5' end of the base sequence of SEQ ID NO: 10 or 11.

**[Table 4]**

| Sensor No. | name | sequence | SEQ ID NO |
|---|---|---|---|
| 10 | N1Mel06_I1_A0_D30_sGC | TGGGTGGGAGGGTCGGGCCCTCCGCTTTTTTGGCTTTTTTTTTTTTGCCTTTTTTGCG | 48 |
| 9 | N1Mel06_I1_A0_D34_sAT | TGGGTGGGAGGGTCGGGCCCTCCGCTTTTTAGGCTTTTTTTTTTTTGCCTTTTTTGCG | 47 |
| 2 | Mel06_dsG | AGGGACGGGAAGAACGCTTTTTTTTTTTTGCGAAAATGTGGAGGGT | 40 |
| 13 | Mel06_dsG_H30 | AGGGACGGGAAGAACGCTTTTTTTTTTTTTTTTTTTTTTTTTTTTTTGCGAAAATGTGGAGGGT | 43 |
| 14 | Mel06_dsG_H36 | AGGGACGGGAAGAACGCTTTTTTTTTTTTTTTTTTTTTTTTTTTTTTTTTTTTTGCGAAAATGTGGAGGGT | 50 |
| 15 | Mel06_dsG_H42 | AGGGACGGGAAGAACGCTTTTTTTTTTTTTTTTTTTTTTTTTTTTTTTTTTTTTTTTTTGCGAAAATGTGGAGGGT | 51 |

Among the polynucleotides (X1) shown in Table 4, each of the polynucleotides each having the base sequence of SEQ ID NO: 48 or 47 includes the base sequence of SEQ ID NO: 12 or 13 and includes an additional sequence of 1 base at the 5' end of the base sequence of SEQ ID NO: 12 or 13. Among the polynucleotides (X1) shown in Table 4, each of the polynucleotides each having the base sequence of SEQ ID NO: 40, 49, 50, or 51 has the bases of SEQ ID NO: 14.

The polynucleotide (X2) has a base sequence obtained by substitution, deletion, addition, and/or insertion of one or more bases in the base sequence of polynucleotide (X1). In the polynucleotide (X2), there is no particular limitation on "one or more", and the above description as to the polynucleotide (x2) can be referred to, for example. As a specific example, in Table 1, the polynucleotide having the bases of SEQ ID NO: 39 includes a sequence in which C at the 5' end of the base sequence of SEQ ID NO: 2 is substituted with T. Specifically, the polynucleotide having the bases of SEQ ID NO: 39 has a sequence in which C at the 5' end of the underlined part on the 3' side of the base sequence of SEQ ID NO: 38 is substituted with T.

The polynucleotide (X3) has a base sequence having at least 80% identity to any base sequence of the polynucleotide (X1). As for the identity, the above description as to the polynucleotide (x2) can be referred to, for example.

The polynucleotide (X4) has a base sequence complementary to a polynucleotide that hybridizes to a polynucleotide having any base sequence of the polynucleotide (X1) under a stringent condition. As for the "hybridizes under a stringent condition", the above description as to the polynucleotide (x4) can be referred to, for example.

The size of the nucleic acid sensor of the present invention is not particularly limited, and the nucleic acid sensor has a length of, for example, 15 to 130 bases, preferably 20 to 110 bases, and more preferably 30 to 90 bases.

The building block of the nucleic acid sensor of the present invention is, for example, a nucleotide residue, and the nucleic acid sensor is, for example, a molecule including the nucleotide residues. The nucleic acid sensor may be a molecule having the nucleotide residues or a molecule including the nucleotide residues. Examples of the nucleotide include ribonucleotides, deoxyribonucleotides, and the derivatives thereof. The nucleic acid sensor may include only one of, two or more of, or all of the ribonucleotides, deoxyribonucleotides, and the derivatives thereof, for example. Specifically, the nucleic acid sensor may be, for example, DNA including deoxyribonucleotides and/or the derivatives thereof, RNA including ribonucleotides and/or the derivatives thereof, or a chimera (DNA/RNA) including both the former and the latter. The nucleic acid sensor is preferably DNA, and can be referred to as, for example, a DNA sensor.

The nucleotide may include either natural bases (non-artificial bases) or unnatural bases (artificial bases) as bases, for example. Examples of the natural base include A, C, G, T, U, and the modified bases thereof. Examples of the modification include methylation, fluoration, amination, and thiation. Examples of the unnatural base include 2'-fluoropyrimidine and 2'-O-methylpyrimidine, and specific examples thereof include 2'-fluorouracil, 2'-aminouracil, 2'-O-methyl uracil, and 2'-thiouracil. The nucleotide may be, for eample, modified nucleotide, and examples of the modified nucleotide include 2'-methylated-uracil nucleotide residue, 2'-methylated-cytosine nucleotide residue, 2'-fluorated-uracil nucleotide residue, 2'-fluorated-cytosine nucleotide residue, 2'-aminated-uracil nucleotide residue, 2'-aminated-cytosine nucleotide residue, 2'-thioated-uracil nucleotide residue, and 2'-thioated-cytosine nucleotide residue. The binding nucleic acid molecule (A) may include non-nucleotide such as a peptide nucleic acid (PNA), a locked nucleic acid (LNA), or the like, for example.

In the nucleic acid sensor of the present invention, for example, the catalytic nucleic acid molecule (D) partially forms a stem with the linker sequence in the absence of melamine and this results in the inhibition of the catalytic function of the catalytic nucleic acid molecule (D). The inhibition of the catalytic function is caused by the caging of the catalytic nucleic acid molecule (D) due to the formation of the stem, for example. In other words, since the catalytic nucleic acid molecule (D) is not allowed to have a structure that activates a catalytic function due to the formation of the stem, the catalytic function is inhibited. On the other hand, in the presence of melamine, the formation of the stem is released by the bond between the melamine and the binding nucleic acid molecule (A), and the catalytic function of the catalytic nucleic acid molecule (D) is activated. The catalytic function is activated when the formation of the stem is released and the caging of the catalytic nucleic acid molecule is released, for example. In other words, since the catalytic nucleic acid molecule (D) is allowed to have an original structure that activates a catalytic function when the formation of the stem is released, the catalytic function is activated. In this manner, in the nucleic acid sensor, the catalytic function of the catalytic nucleic acid molecule (D) is inhibited (switch OFF) due to the formation of the stem in the absence of melamine, and the catalytic function of the catalytic nucleic acid molecule (D) is activated (switch ON) when the formation of the stem is released in the presence of melamine, for example. Note here that, the present invention is not limited to these mechanisms.

The nucleic acid sensor of the present invention may be used in a free state or in an immobilized state, for example. In the latter case, for example, the nucleic acid sensor can be used as a device by immobilizing it on the base material. Examples of the base material include base boards such as plates, sheets, films, and swabs; containers such as well plates and tubes; beads; particles; and filters. The nucleic acid sensor may be immobilized with either the 5' end or the 3' end, for example.

The method of immobilization is not particularly limited, and an example thereof includes a linkage by a chemical bond. As a specific example, there is a method in which streptavidin or avidin is caused to bind to either one of the carrier and the nucleic acid sensor and biotin is caused to bind to the other, and the bond between the former and the latter is utilized for immobilization.

In addition to this, as the method of immobilization, a well known nucleic acid immobilization method can be employed, for example. An example of the method includes a method of utilizing photolithography, and a specific example thereof includes the specification of U.S. Pat. No. 5,424,186. Furthermore, an example of the method of immobilization includes a method in which the nucleic acid sensor is synthesized on the base material. An example of this method includes a so-called spot method, and specific examples thereof include the specifications of U.S. Pat. No. 5,807,522 and JP 10(1998)-503841.

The nucleic acid sensor may be immobilized on the base material either directly or indirectly, for example. In the former case, preferably, the nucleic acid sensor is immobilized on the base material at the end of the nucleic acid sensor, for example. In the latter case, the nucleic acid sensor may be immobilized on the base material via a linker sequence for immobilization, for example. With respect to the arrangement of the nucleic acid sensor on the base material, for example, see the description as to the analysis device of the present invention that will be described below.

The usage of the nucleic acid sensor of the present invention is not particularly limited, and the nucleic acid sensor of the present invention can be used for the method for melamine analysis of the present invention.

As described above, the analysis method of the present invention is a method for melamine analysis including: a contact step of bringing a sample into contact with the nucleic acid sensor according to the present invention; and a detection step of detecting the catalytic function of the catalytic nucleic acid molecule (D) in the nucleic acid sensor to detect melamine in the sample.

The sample is not particularly limited. The sample may be, for example, either a sample containing melamine or a sample possibly containing melamine. Preferably, the sample is, for example, a liquid sample. When a test object is, for example, a liquid object, the test object may be used as a sample without processing or a diluted solution obtained by mixing the test object in a solvent may be used as a sample. When the test object is, for example, a solid object, a powder object, or the like, a mixture obtained by mixing the test object in a solvent, a suspension obtained by suspending the test object in a solvent, or the like may be used as a sample. The solvent is not particularly limited, and examples thereof include water and buffer solutions. Specific examples of the test object include raw milk, processed milk, and milk powder.

When the nucleic acid sensor of the present invention is used in a free state, it is preferable to bring the sample into contact with the nucleic acid sensor in the container, for example. Furthermore, when the nucleic acid sensor of the present invention is used in the state where it is immobilized on the base material, it is possible to bring the sample into contact with the nucleic acid sensor on the base material, for example.

Preferably, the detection step detects a signal produced by the catalytic function of the catalytic nucleic acid molecule (D), for example. Examples of the signal include optical signals and electrochemical signals. Examples of the optical signal include color development signals, luminescent signals, and fluorescent signals.

Preferably, the signal is produced from a substrate by the catalytic function of the catalytic nucleic acid molecule (D), for example. Hence, preferably, the detection step is performed in the presence of a substrate appropriate to the catalytic function of the catalytic nucleic acid molecule (D), for example.

Examples of the substrate include a substrate that produces a color development product, a luminescent product, or a fluorescent product by the catalytic function; a substrate that produces a product quenching its color development, luminescence, or fluorescence by the catalytic function; and a substrate that produces a product changing its color development, luminescence, or fluorescence by the catalytic function. Such substrates allow to detect the catalytic function by visually examining the presence or absence of the color development, the luminescence, or the fluorescence or the change, the intensity, or the like of the color development, the luminescence, or the fluorescence as a signal, for example. Furthermore, for example, by measuring the absorbance, the reflectance, the fluorescence intensity, or the like as a signal by an optical method, the catalytic function can be detected. An example of the catalytic function includes the above-described catalytic function of a redox reaction.

Furthermore, in the case where the catalytic nucleic acid molecule (D) has the catalytic function of the redox reaction, an example of the substrate includes a substrate that allows electron transfer. In this case, for example, a product is produced from the substrate by the catalytic nucleic acid molecule (D), and electron transfer occurs in that process. For example, this electron transfer can be electrochemically detected as an electrical signal by applying voltage to electrodes. The electrical signal can be detected by measuring the intensity of the electrical signal such as a current or the like, for example.

The substrate is not particularly limited, and examples thereof include hydrogen peroxide, 3,3',5,5'-Tetramethylbenzidine (TMB), 1,2-Phenylenediamine (OPD), 2,2'-Azinobis(3-ethylbenzothiazoline-6-sulfonic Acid Ammonium Salt (ABTS), 3,3'-Diaminobenzidine (DAB), 3,3'-Diaminobenzidine Tetrahydrochloride Hydrate (DAB4HCl), 3-Amino-9-ethylcarbazole (AEC), 4-Chloro-1-naphthol (4ClN), 2,4,6-Tribromo-3-hydroxybenzoic Acid, 2,4-Dichlorophenol, 4-Aminoantipyrine, 4-Aminoantipyrine Hydrochloride, and luminol.

In the detection step, for example, the substrate may be preliminarily supplied to the nucleic acid sensor before bringing the sample into contact with the nucleic acid sensor or supplied at the same time as or after bringing the sample into contact with the nucleic acid sensor. The substrate is preferably supplied to the nucleic acid sensor as a substrate liquid obtained by mixing the substrate in a liquid, for example. Examples of the liquid in which the substrate is mixed to obtain the substrate liquid include buffer solutions such as Tris-HCl and the like. The concentration of the substrate in the substrate liquid is not particularly limited, and the concentration is, for example, 0.1 to 5 mmol/L and preferably 0.5 to 2 mmol/L. Furthermore, pH of the substrate liquid is, for example, 6 to 9 and is preferably 6.8 to 9.

In the detection step, there is no particular limitation on conditions for the reaction by the catalytic nucleic acid molecule (D). The temperature is, for example, 15 to 37°C and the time is, for example, 10 to 900 seconds.

In the detection step, for example, porphyrin may coexist in addition to the substrate. For example, some of the well known DNAzymes show higher redox activity by forming a complex with porphyrin. Hence, also in the present invention, the redox activity may be detected by causing porphyrin to coexist to form a complex of the catalytic nucleic acid molecule (D) and porphyrin, for example. There is no particular limitation on the supply of the porphyrin, and can be supplied in the same manner as the substrate.

The porphyrin is not particularly limited, and examples thereof include unsubstituted porphyrins and the derivatives thereof. Examples of the derivatives include substituted porphyrins and metal porphyrins that formed complexes with metal elements. An example of the substituted porphyrin includes N-Methylmesoporphyrin. An example of the metal porphyrin includes hemin, which is a ferric complex. For example, the porphyrin is preferably the metal porphyrin and is more preferably hemin.

The analysis method of the present invention may further include a washing step between the contact step and the detection step. The washing step is, for example, a step of washing the nucleic acid sensor with a washing liquid after bringing the sample into contact with the nucleic acid sensor. The washing step allows impurities contained in the sample to be removed and allows an analysis with excellent accuracy, for example. The washing liquid is not particularly limited, and examples thereof include aqueous solvents such as water and buffer solutions. The nucleic acid sensor is preferably immobilized on the base material because it allows the washing step to be performed easily, for example.

### (Analysis device and analysis method using the same)

The analysis device of the present invention is a device for melamine analysis including: a base material; a nucleic acid sensor; and a detection unit, wherein the nucleic acid sensor and the detection unit are arranged on the base material, the nucleic acid sensor is the nucleic acid sensor according to the present invention, and the detection unit is a detection unit detecting the catalytic function of the catalytic nucleic acid molecule (D) in the nucleic acid sensor.

The analysis device of the present invention is characterized in that it includes the nucleic acid sensor of the present invention, and other configurations are not limited by any means. Unless otherwise stated, the above description as to the nucleic acid sensor of the present invention can be referred to in order to understand the analysis device of the present invention, for example.

In the analysis device of the present invention, the method for arranging the nucleic acid sensor is not particularly limited. In the analysis device, the nucleic acid sensor may or may not be immobilized on the base material, for example. Regarding the arrangement of the nucleic acid sensor, the above description as to the nucleic acid sensor of the present invention can be referred to, for example.

The position at which the nucleic acid sensor is arranged on the base material is not particularly limited. For example, the nucleic acid sensor may be arranged in the detection unit.

The analysis device of the present invention may further include a reagent unit, for example. The reagent unit may be arranged in the detection unit, for example. A reagent may be arranged in the reagent unit in advance or may be supply to the reagent unit at the time of using the analysis device, for example. Examples of the reagent include substrates as defined above and the above-described porphyrins.

In the analysis device of the present invention, the detection unit is, as described above, a detection unit detecting the catalytic function of the catalytic nucleic acid molecule (D). Preferably, the detection unit detects a signal produced by the catalytic function of the catalytic nucleic acid molecule (D), for example. The signal may be, as described above, produced from a substrate by the catalytic function of the catalytic nucleic acid molecule (D), for example. The signal may be, for example, an optical signal or an electrochemical signal as described above. The detection unit also can be referred to as, for example, a "detected unit" because a signal produced in the detection unit is detected from the outside.

When the signal is an optical signal, the detection unit may be an optical signal detection unit, for example. The optical signal detection unit may be, for example, a detection unit for detection of absorbance, reflectance, fluorescence intensity, or the like.

When the signal is the electrochemical signal, the detection unit includes an electrode system, for example. In this case, the detection unit can be formed by arranging the electrode system on a surface of the base material, for example. The method for arranging the electrodes is not particularly limited, and a known method can be employed, for example. Specific examples of the method include thin film forming methods such as vapor deposition, sputtering, screen printing, and plating. The electrodes may be arranged on the base material either directly or indirectly, for example. When the electrodes are arranged on the base material indirectly, they may be arranged via another member, for example.

The electrode system may include a working electrode and a counter electrode, or may include a working electrode, a counter electrode, and a reference electrode, for example. The material of each electrode is not particularly limited, and examples thereof include platinum, silver, gold, and carbon. The working electrode and the counter electrode each may be a platinum electrode, a silver electrode, a gold electrode, a carbon electrode, or the like, for example. The reference electrode may be a silver/silver chloride electrode, for example. The silver/silver chloride electrode can be formed by laminating a silver chloride electrode on a silver electrode, for example.

When the analysis device of the present invention includes the electrode system, the nucleic acid sensor preferably is arranged in the electrode system, for example. Among the electrodes, it is preferable that the nucleic acid sensor is arranged in the working electrode. When the analysis device of the present invention includes the electrode system and the reagent unit, the reagent unit preferably is arranged on the electrode system, for example.

The analysis device of the present invention may include a plurality of detection units, for example. In this case, the analysis device preferably is configured so that, for example, the surface of the base material is divided in a matrix form, and the above-described detection units are provided in the respective divided regions. In the analysis device of the present invention, the number of nucleic acid sensors to be arranged in a single detection unit is not particularly limited.

The base material is not particularly limited. Preferably, the base material is a base board having an insulating surface(s), for example. For example, the base material may be a base board formed of an insulating material, or the base material may have an insulating layer formed of an insulating material on a surface thereof. The insulating material is not particularly limited, and may be, for example, a known material such as glass, ceramic, insulating plastic, paper, or the like. The insulating plastic is not particularly limited, and may be, for example, a silicone resin, a polyimide resin, an epoxy resin, a fluororesin, or the like.

The analysis method of the present invention is, as described above, a method for melamine analysis including: a contact step of bringing a sample into contact with the device for analysis according to the present invention; and a detection step of detecting the catalytic function of the catalytic nucleic acid molecule (D) in the detection unit of the device for analysis to detect melamine in the sample.

The analysis method of the present invention is characterized in that it uses the analysis device provided with the nucleic acid sensor of the present invention, and other conditions are not limited by any means. Regarding the analysis method of the present invention, the description as to the analysis method in the above description as to the nucleic acid sensor of the present invention can be referred to, for example.

### (Analysis reagent)

The analysis reagent of the present invention is characterized in that it includes the analysis nucleic acid sensor of the present invention. The analysis reagent of the present invention is characterized in that it includes the nucleic acid sensor, and other configurations are not limited by any means.

The analysis reagent of the present invention may include, in addition to the nucleic acid sensor, a component(s) such as the substrate, the porphyrin, the buffer solution, and/or the base material, for example.

The analysis reagent of the present invention may be an analysis kit, for example. In this case, for example, the analysis kit includes the nucleic acid sensor and the above-described other component(s), and they may be contained in separate containers, respectively. The nucleic acid sensor may or may not be immobilized on the base material, for example. The analysis kit may further include instructions for use, for example.

### Examples

### (Example 1)

The present example examined the effect of the length of the additional sequence for immobilization in nucleic acid sensors. Specifically, nucleic acid sensors having additional sequences for immobilization with different lengths were used in a free state, and the melamine-detecting abilities of these sensors in the free state were examined. In the following sequences, the left side is the 5' end and the right side is the 3' end (the same applies hereinafter).

### (1) Nucleic acid sensors

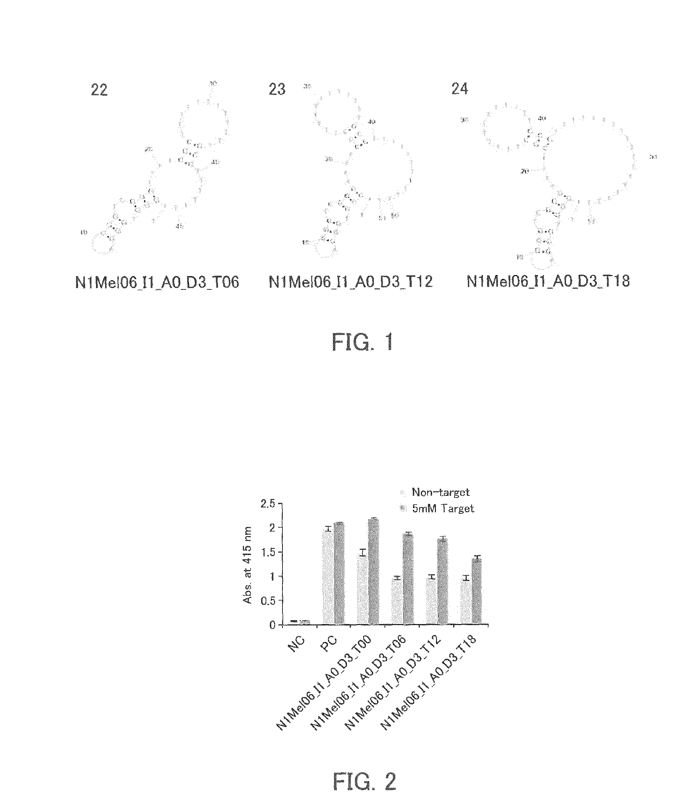

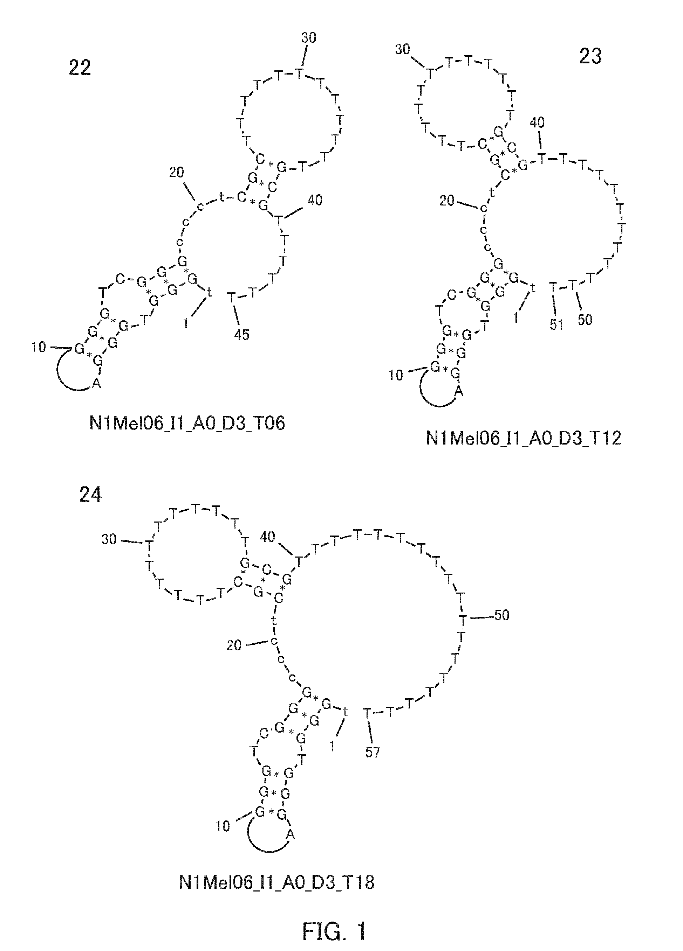

The following nucleic acid sensors were produced: nucleic acid sensors shown in Table 5 below, each having a melamine aptamer Mel06 (SEQ ID NO: 57, n = 12) as the binding nucleic acid molecule (A) and a DNAzyme neco0584 (SEQ ID NO: 61) as the catalytic nucleic acid molecule (D). In the sequences shown in Table 5, poly(dT) sequences on the 3'-end side are the additional sequences for immobilization. In each of the sequences, the underlined part on the 5' side is the DNAzyme, and the underlined part on the 3' side is the melamine aptamer. The secondary structures of these nucleic acid sensors are shown in FIG. 1. The predicted secondary structures shown in FIG 1 are each in an active form in the presence of melamine.

**[Table 5]**

| Sensor No. | name | sequence | SEQ ID NO |
|---|---|---|---|
| 0 | N1Mel06_I1_A0_D3_T00 | tGGGTGGGAGGGTCGGGccctCGCTTTTTTTTTTTTGCG | 15 |
| 22 | N1Mel06_I1_A0_D3_T06 | tGGGTGGGAGGGTCGGGccctCGCTTTTTTTTTTTTGCGtttttt | 16 |
| 23 | N1Mel08_I1_A0_D3_T12 | tGGGTGGGAGGGTCGGGccctCGCTTTTTTTTTTTTGCGtttttttttttt | 17 |
| 24 | N1Mel06_I1_A0_D3_T18 | tGGGTGGGAGGGTCGGGccctCGCTTTTTTTTTTTTGCGtttttttttttttttttt | 18 |

### (2) Colorimetric analysis

Respective components were added to wells of a microwell plate so that the resultant mixture had the following composition.

### (Composition in each well)

| | |
|---|---|
| 1 µmol/L | nucleic acid sensor |
| 3 µmol/L | hemin |
| 50 mmol/L | Tris-HCl (pH 7.4) |
| 20 mmol/L | KCl |
| 0.05% (w/v) | Triton X-100 |

Next, melamine was added to each well at a concentration of 5 µmol/L, and further, ABTS (2,2'-azinobis(3-ethylbenzothiazoline-6-sulfonic acid ammonium salt) was added as a substrate so as to achieve a concentration of 20 mmol/L, and H₂O₂ was added so as to achieve a concentration of 0.5 mmol/L. The thus-obtained reaction solution was reacted at 25°C for 60 seconds, and the absorbance thereof was measured (at a wavelength of 415 nm). The measurement was carried out using a measurement device (trade name: TECAN infinite F200 plate reader, manufactured by TECAN).

As a positive control (PC), the measurement was carried out in the same manner, except that the DNAzyme neco0584 (SEQ ID NO: 61) was used instead of the nucleic acid sensor. Also, as a negative control (NC), the measurement was carried out in the same manner with respect to a reaction solution excluding the nucleic acid sensor (without oligo).

The results thereof are shown in FIG. 2. FIG. 2 is a graph showing the absorbances of the reaction solutions. As can be seen from FIG. 2, it was verified that the nucleic acid sensors can detect melamine even if the length of the additional sequence for immobilization is changed. Furthermore, the nucleic acid sensors having the additional sequences on their 3' side exhibited higher S/N ratios than the nucleic acid sensor having no additional sequence.

### (Example 2)

The present example examined the melamine-detecting ability of nucleic acid sensors in a free state.

### (1) Nucleic acid sensors

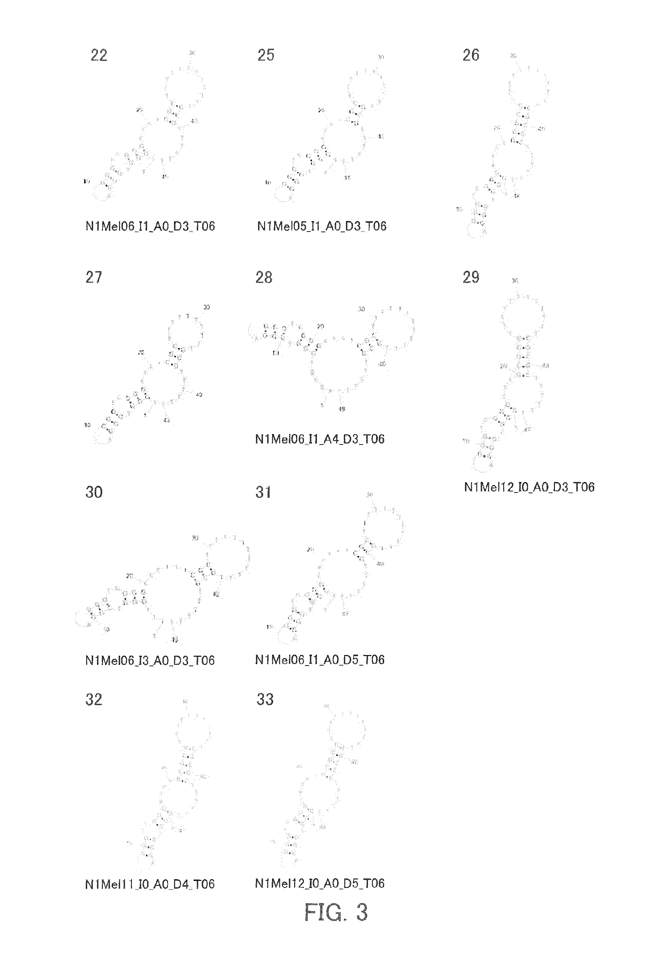

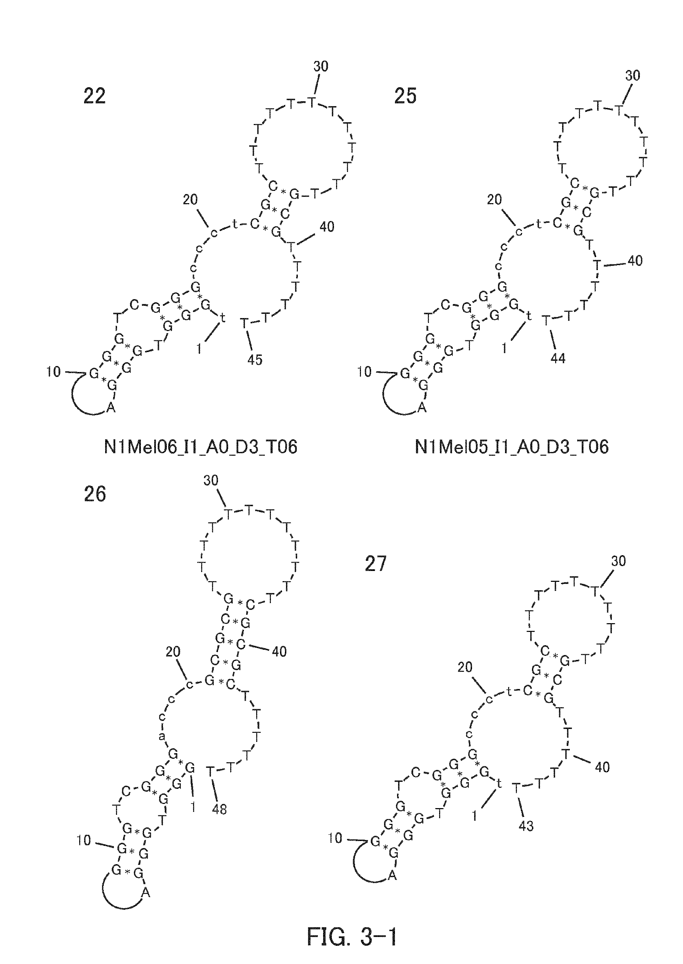

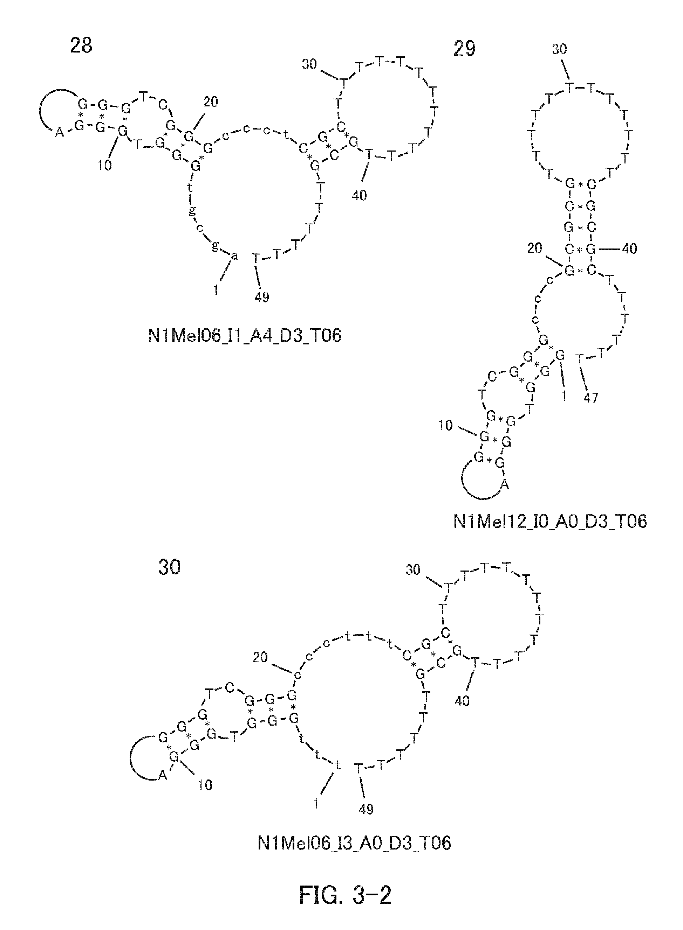

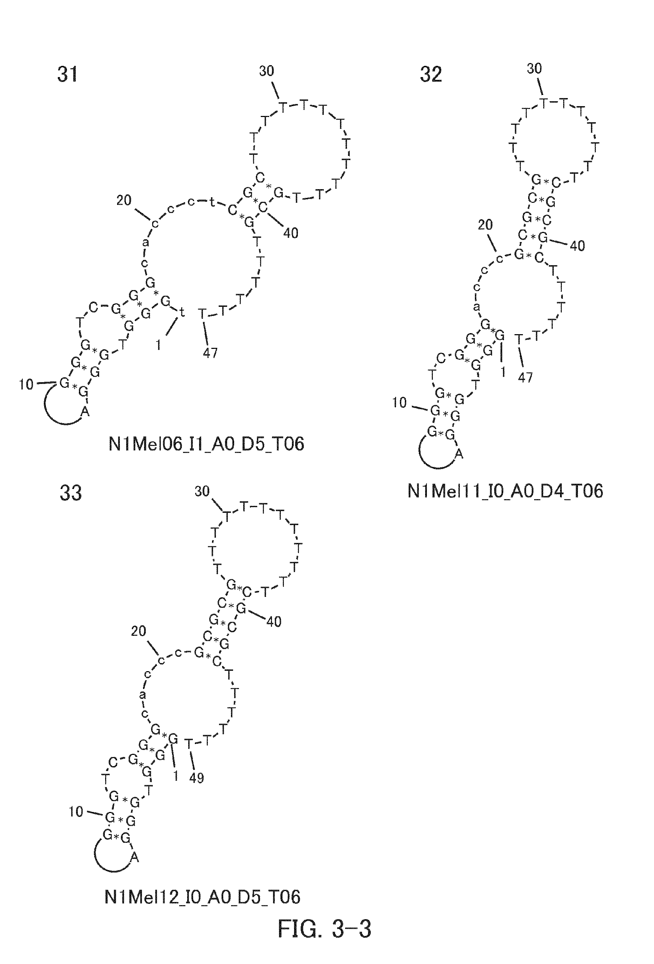

The following nucleic acid sensors were produced: nucleic acid sensors shown in Table 6 below, having melamine aptamers Mel04 (SEQ ID NO: 57, n = 10), Mel05 (SEQ ID NO: 57, n = 11), Mel06 (SEQ ID NO: 57, n = 12), Mel11 (SEQ ID NO: 58, n = 11), and Mel12 (SEQ ID NO: 58, n = 12), respectively, as the binding nucleic acid molecule (A) and a DNAzyme neco0584 (SEQ ID NO: 61) as the catalytic nucleic acid molecule (D). In each of the sequences shown in Table 6, the poly(dT) sequence on the 3'-end side is the additional sequence for immobilization. In the sequence, the underlined part on the 5' side is the DNAzyme, and the underlined part on the 3' side is the melamine aptamer. The secondary structures of these nucleic acid sensors are shown in FIG 3. The predicted secondary structures shown in FIG. 3 are each in an active form in the presence of melamine.

**[Table 6]**

| Sensor No. | name | sequence | SEQ ID NO |
|---|---|---|---|
| 22 | N1Mel06_I1_A0_D3_T06 | tGGGTGGGAGGGTCGGGccctCGCTTTTTTTTTTTTGCGtttttt | 16 |
| 25 | N1MelD5_I1_AD_D3_T06 | tGGGTGGGAGGGTCGGGccctCGCTTTTTTTTTTTGCGtttttt | 19 |
| 26 | N1Mel12_I0_A0_D4_T06 | GGGTGGGAGGGTCGGGacccGCGCGTTTTTTTTTTTTCGCGCtttttt | 20 |
| 27 | N1Mel04_I1_A0_D3_T06 | tGGGTGGGAGGGTCGGGccctCGCTTTTTTTTTTGCGtttttt | 21 |
| 28 | N1Mel08_I1_A4_D3_T08 | agcgtGGGTGGGAGGGTCGGGccctCGCTTTTTTTTTTTTGCGtttttt | 22 |
| 29 | N1Mel12_I0_A0_03_T06 | GGGTGGGAGGGTCGGGcccGCGCGTTTTTTTTTTTTGCGtttttt | 23 |
| 30 | N1Mel06_I3_A0_D3_T06 | tttGGGTGGGAGGGTCGGGccctttCGCTTTTTTTTTTTTGCGtttttt | 24 |
| 31 | N1Mel06_I1_A0_D5_T06 | tGGGTGGGAGGGTCGGGcaccctCGCTTTTTTTTTTTTGCGtttttt | 25 |
| 32 | N1Mel11_I0_A0_D4_T06 | GGGTGGGAGGGTCGGGacccGCGCGTTTTTTTTTTTTCGCGCtttttt | 26 |
| 33 | N1Mel12_I0_A0_D5_T06 | GGGTGGGAGGGTCGGGcacccGCGCGTTTTTTTTTTTTCGCGCtttttt | 27 |

### (2) Colorimetric analysis

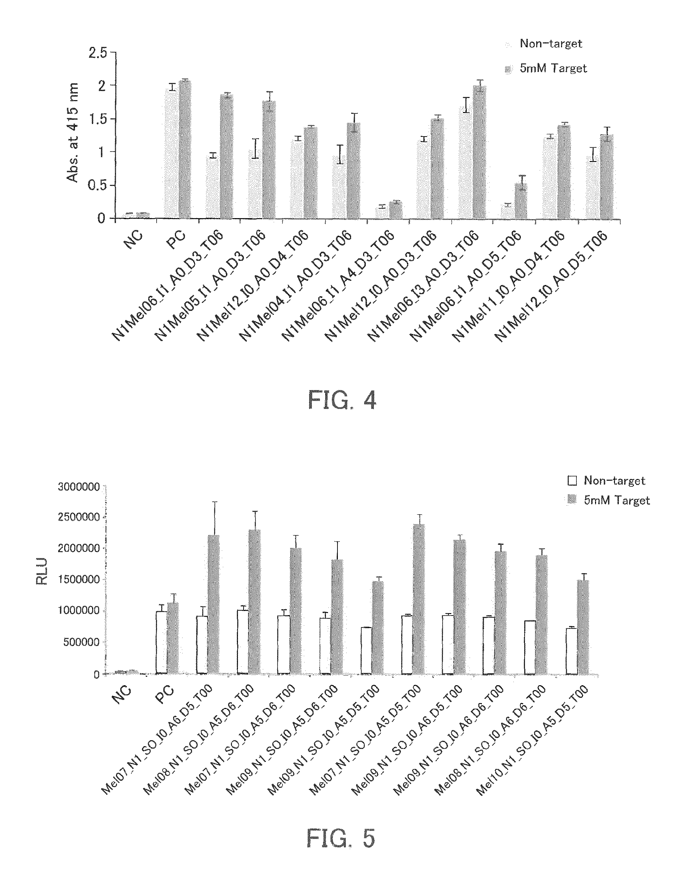

The absorbances of the respective nucleic acid sensors were measured in the same manner as in Example 1. The results thereof are shown in FIG. 4. FIG. 4 is a graph showing the absorbances of the reaction solutions. As can be seen from FIG. 4, it was revealed by the non-parametric two-tailed t-tests that, when the nucleic acid sensors of the present example were used, the absorbances of the reaction solutions containing melamine were significantly different from those of the reaction solutions containing no melamine. In particular, when N1Mel06_I1_A0_D5_T06 was used, the absorbance of the reaction solution containing the target was 2.8 times higher than that of the reaction solution not containing the target. These results verify that, according to the nucleic acid sensors of the present example, it is possible to determine the presence or absence of melamine and to measure the concentration of melamine by absorbance measurement, and specifically, the nucleic acid sensors can detect melamine even when the concentration thereof is 5 mmol/L.

### (Example 3)

The present example examined the melamine-detecting ability of nucleic acid sensors in a free state.

### (1) Nucleic acid sensors

The following nucleic acid sensors were produced: nucleic acid sensors having melamine aptamers Mel07 (SEQ ID NO: 58, n = 7), Mel08 (SEQ ID NO: 58, n = 8), Mel09 (SEQ ID NO: 58, n = 9), and Mel10 (SEQ ID NO: 58, n = 10), respectively, as the binding nucleic acid molecule (A), and a DNAzyme neco0584 (SEQ ID NO: 61) as the catalytic nucleic acid molecule (D). In each of the following sequences, the underlined part on the 5' side is the melamine aptamer, and the underlined part on the 3' side is the DNAzyme.

**[Table 7]**

| name | sequence | SEQ ID NO |
|---|---|---|
| Mel07_N1_S0_I0_A6_D5_T00 | CACCCGCGCGTTTTTTTCGCGCACGCGCGGGTGGGAGGGTCGGG | 28 |
| Mel0B_N1_S0_I0_A5_D6_T00 | CCACCCGCGCGTTTTTTTTCGCGCCGCGCGGGTGGGAGGGTCGGG | 29 |
| Mel07_N1_S0_I0_A5_D6_T00 | CCACCCGCGCGTTTTTTTCGCGCCGCGCGGGTGGGAGGGTCGGG | 30 |
| Mel09_N1_S0_I0_A5_D6_T00 | CCACCCGCGCGTTTTTTTTTCGCGCCGCGCGGGTGGGAGGGTCGGG | 31 |
| Mel09_N1_S0_I0_A5_D5_T00 | CACCCGCGCGTTTTTTTTTCGCGCCGCGCGGGTGGGAGGGTCGGG | 32 |
| Mel07_N1_S0_I0_A5_D5_T00 | CACCCGCGCGTTTTTTTCGCGCCGCGCGGGTGGGAGGGTCGGG | 33 |
| Mel09_N1_S0_I0_A6_D5_T00 | CACCCGCGCGTTTTTTTTTCGCGCACGCGCGGGTGGGAGGGTCGGG | 34 |
| Mel09_N1_S0_I0_A6_D6_T00 | CCACCCGCGCGTTTTTTTTTCGCGCACGCGCGGGTGGGAGGGTCGGG | 35 |
| Mel08_N1_S0_I0_A6_D6_T00 | CCACCCGCGCGTTTTTTTTCGCGCACGCGCGGGTGGGAGGGTCGGG | 36 |
| Mel10_N1_S0_I0_A5_D5_T00 | CACCCGCGCGTTTTTTTTTTCGCGCCGCGCGGGTGGGAGGGTCGGG | 37 |

### (2) Chemiluminescence analysis

To an Eppendorf tube, the following reagent 1, reagent 2, and reagent 3 were added in this order, and the mixture was reacted at 25°C for 60 seconds. Thereafter, the relative chemiluminescence intensity (RLU (relative light unit)) of the reaction solution was measured. Each concentration in the following compositions indicates the final concentration in the reaction solution (the same applies hereinafter). The measurement was carried out using a measurement device (trade name: TECAN infinite, manufactured by TECAN). As a substrate, L-012 (Wako Pure Chemical Industries, Ltd.), which is a luminol derivative, was used.

| (Reagent 1) | |
|---|---|
| 400 nmol/L | nucleic acid sensor |
| 200 nmol/L | hemin |
| 50 mmol/L | Tris-HCl (pH 7.4) |
| 20 mmol/L | KCl |
| 0.05% (w/v) | Triton X-100 |

| (Reagent 2) | |
|---|---|
| 5 mmol/L | melamine |

| (Reagent 3) | |
|---|---|
| 25 µmol/L | L-012 |
| 25 µmol/L | H₂O₂ |

As a positive control (PC), the measurement was carried out in the same manner, except that the DNAzyme neco0584 (SEQ ID NO: 61) was used instead of the nucleic acid sensor. Also, as a negative control (NC), the measurement was carried out in the same manner with respect to a reaction solution excluding the nucleic acid sensor. The same PC and NC were used in the following examples.

The results thereof are shown in FIG. 5. FIG. 5 is a graph showing the luminescent intensities (RLU) of the reaction solutions. As can be seen from FIG. 5, when the nucleic acid sensors of the present example were used, the luminescence intensities of the reaction solutions containing melamine were significantly different from those of the reaction solutions containing no melamine. From these results, it was found that, according to the nucleic acid sensors of the present example, it is possible to determine the presence or absence of melamine and to measure the concentration of melamine by luminescence intensity measurement.

Table 8 shows nucleic acid sensors used in Examples 4 to 9 to be described below.

**[Table 8]**

| name | sequence | SEQ ID NO |
|---|---|---|
| N1Mel06_I1_A0_D3_T00_C | TGGGTGGGAGGGTCGGGCCCTCCGCTTTTTTTTTTTTGCG | 38 |
| N1Mel08_I1_A0_D3_T00_T | TGGGTGGGAGGGTCGGGCCCTCTGCTTTTTTTTTTTTGCG | 39 |
| Mel06_dsG | AGGGACGGGAAGAACGCTTTTTTTTTTTTGCGAAATGTGGAGGGT | 40 |
| N1Mel06_I1_A0_D3C_1 | GGGTGGGAGGGTCGGGCCCTCCGCTTTTTTTTTTTTGCG | 41 |
| N1Mel06_I1_A0_D3C_C | TGGGTGGGAGGGTCGGGCCCTCCCGCTTTTTTTTTTTTGCG | 42 |
| N1Mel06_I1_A0_D3C_CG | TGGGTGGGAGGGTCGGGCCCTCCCGCTTTTTTTTTTTTGCGG | 43 |
| N1Mel06_I1_A0_D3C_H24 | TGGGTGGGAGGGTCGGGCCCTCCGCTTTTTTTTTTTTTTTTTTTTTTTTTGCG | 44 |
| N1Mel06_I1_A0_D3C_H36 | TGGGTGGGAGGGTCGGGCCCTCCGCTTTTTTTTTTTTTTTTTTTTTTTTTTTTTTTTTTTTTGCG | 45 |
| N1Mel06_I1_A0_D3C_H48 | TGGGTGGGAGGGTCGGGCCCTCCGCTTTTTTTTTTTTTTTTTTTTTTTTTTTTTTTTTTTTTTTTTTTTTTTTTGCG | 46 |
| N1Mel06_I0_A0_D3C_sAT | TGGGTGGGAGGGTCGGGCCCTCCGCTTTTTAGGCTTTTTTTTTTTTGCCTTTTTTGCG | 47 |
| N1Mel06_I1_A0_D3C_sGC | TGGGTGGGAGGGTCGGGCCCTCCGCTTTTTTGGCTTTTTTTTTTTTGCCTTTTTTGCG | 48 |
| Mel06_dsG_H30 | AGGGACGGGAAGAACGCTTTTTTTTTTTTTTTTTTTTTTTTTTTTTTGCGAAATGTGGAGGGT | 49 |
| Mel06_dsG_H36 | AGGGACGGGAAGAACGCTTTTTTTTTTTTTTTTTTTTTTTTTTTTTTTTTTTTGCGAAATGTGGAGGGT | 50 |
| Mel06_dsG_H42 | AGGGACGGGAAGAACGCTTTTTTTTTTTTTTTTTTTTTTTTTTTTTTTTTTTTGCGAAATGTGGAGGGT | 51 |
| H1Mel06_I1_A0_D3C_CG_H30 | TGGGTGGGAGGGTCGGGCCCTCCCGCTTTTTTTTTTTTTTTTTTTTTTTTTTTTTTGCGG | 52 |
| N1Mel06_I1_A0_D3C_CG_H36 | TGGGTGGGAGGGTCGGGCCCTCCCGCTTTTTTTTTTTTTTTTTTTTTTTTTTTTTTTTTTTTGCGG | 53 |
| N1Mel08_I1_A0_D8C_CG_H42 | TGGGTGGGAGGGTCGGGCCCTCCCGCTTTTTTTTTTTTTTTTTTTTTTTTTTTTTTTTTTTTTTTTTTGCGG | 54 |
| N1Mel06_I1_A0_D3C_H30 | TGGGTGGGAGGGTCGGGCCCTCCCGCTTTTTTTTTTTTTTTTTTTTTTTTTTTTTTGCGG | 55 |
| N1Mel06_I1_A0_D3C_H42 | TGGGTGGGAGGGTCGGGCCCTCCCGCTTTTTTTTTTTTTTTTTTTTTTTTTTTTTTTTTTTTTTTTTTGCGG | 56 |

### (Example 4)

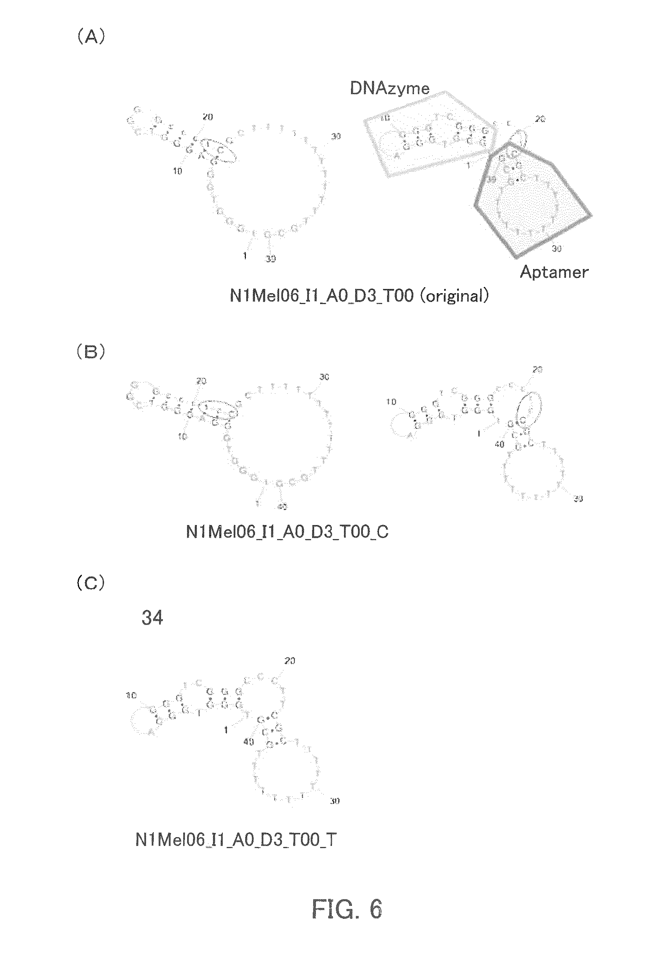

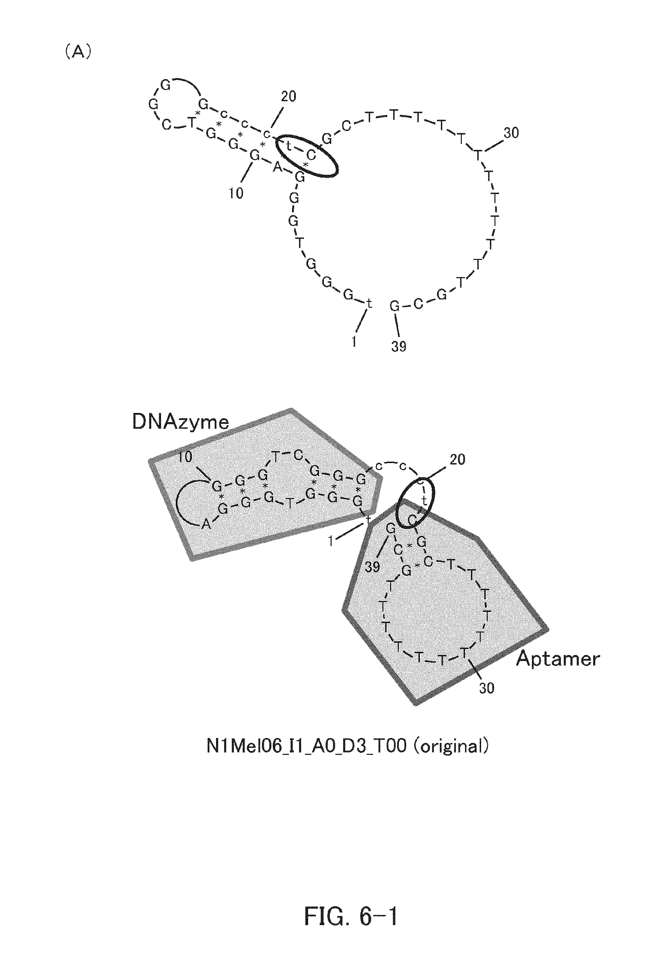

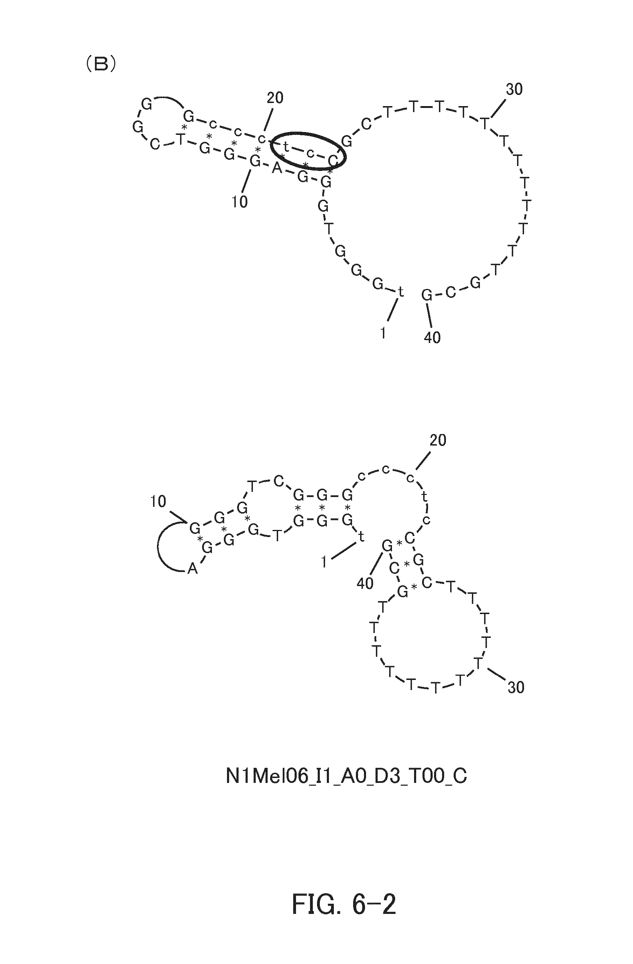

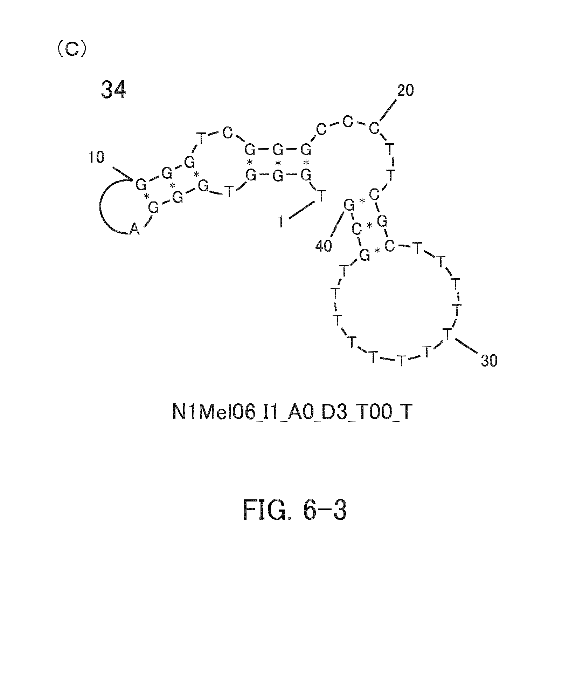

FIG. 6A shows the predicted secondary structures of N1Mel06_I1_A0_D3_T00 (SEQ ID NO: 15), which is one of the nucleic acid sensors used in Example 1. In FIG. 6A, the structure on the left is the predicted secondary structure in a non-active form in the absence of melamine, and the structure on the right is the predicted secondary structure in an active form in the presence of melamine.

This N1Mel06_I1_A0_D3_T00 was modified by further inserting dC between the 21st dT (T21) and the 22th dC (C22) in the internal loop, whereby N1Mel06_I1_A0_D3_T00_C (SEQ ID NO: 38) was produced. FIG. 6B shows the predicted secondary structure of N1Mel06_I1_A0_D3_T00_C. In FIG. 6B, the structure on the left is the predicted secondary structure in a non-active form in the absence of melamine, and the structure on the right is the predicted secondary structure in an active form in the presence of melamine. Also, N1Mel06_I1_A0_D3_T00_T (SEQ ID NO: 39) was produced, in which dT was insert instead of dC. FIG. 6C shows the predicted secondary structure of N1Mel06_I1_A0_D3_T00_T. The predicted secondary structure shown in FIG. 6C is in an active form in the presence of melamine.

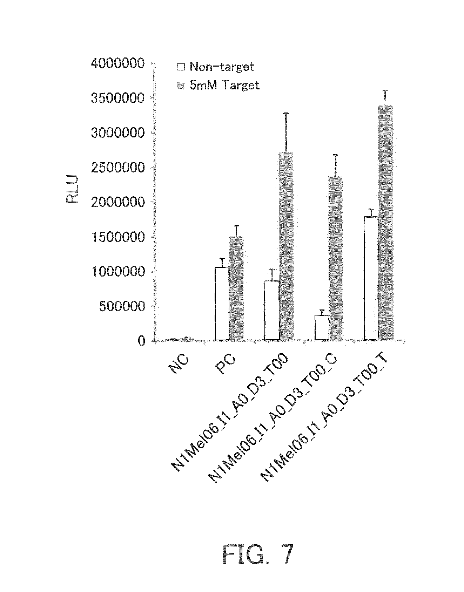

The relative chemiluminescence intensities (RLU) of the above nucleic acid sensors were measured in the same manner as in Example 3. The results thereof are shown in FIG. 7. FIG. 7 is a graph showing the luminescence intensities (RLU) of the reaction solutions. As can be seen from FIG. 7, these sensors all could detect melamine. In particular, N1Mel06_I1_A0_D3_T00_C (SEQ ID NO: 38), which further includes dC inserted therein, exhibited a still further improved ratio (S/N) between the luminescence intensity in the presence of melamine and the luminescence intensity in the absence of melamine. Specifically, while the S/N exhibited by N1Mel06_I1_A0_D3_T00 (SEQ ID NO: 15) was 3.2, the S/N exhibited by N1Mel06_I1_A0_D3_T00_C (SEQ ID NO: 38) was further improved to 6.5.

### (Example 5)

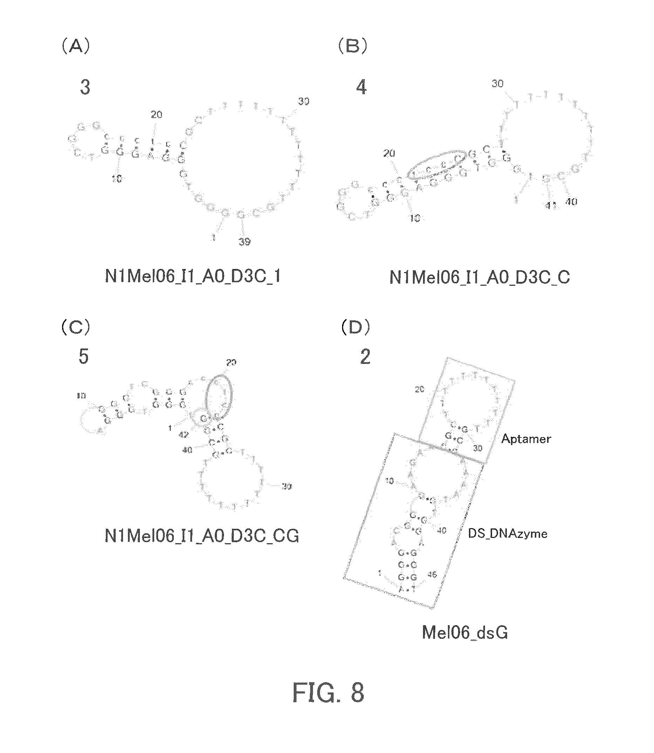

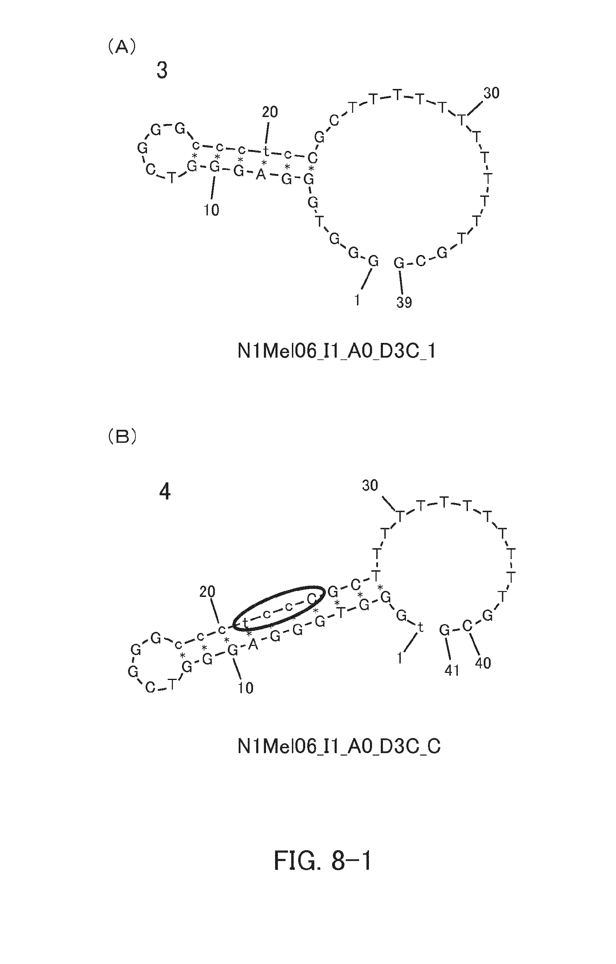

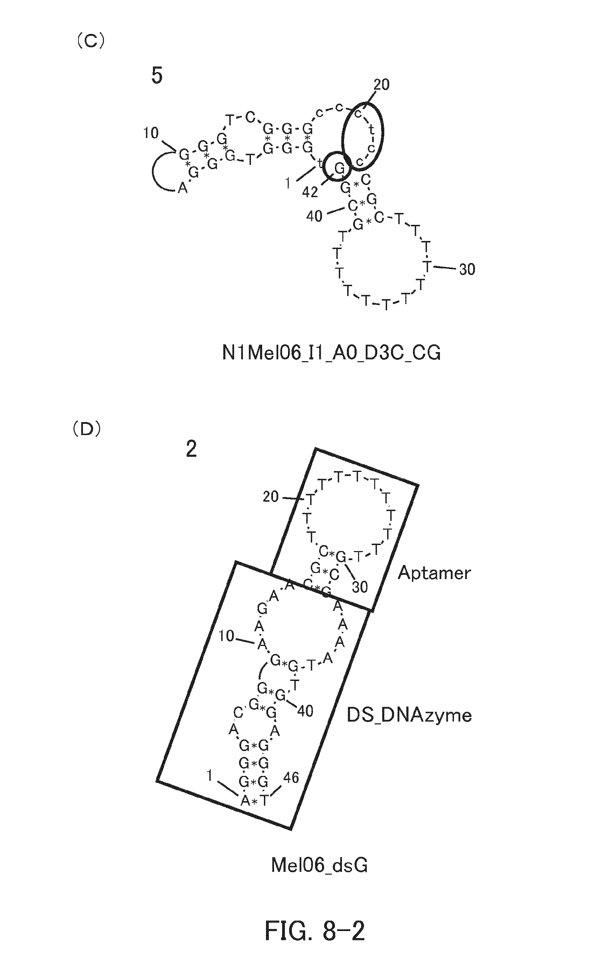

Modified sensors were produced by modifying the nucleic acid sensor N1Mel06_I1_A0_D3_T00_C (SEQ ID NO: 38) of Example 4. Specifically, in the predicted secondary structure of N1Mel06_I1_A0_D3_T00_C shown in FIG. 6B, dT at the 5' end was deleted to produce N1Mel06_I1_A0_D3C_1 (SEQ ID NO: 41), and dC was inserted between the 21st dT (T21) and the 22nd dC (C22) to produce N1Mel06_I1_A0_D3C_C (SEQ ID NO: 42). The predicted secondary structures thereof are shown in FIGs. 8A and 8B, respectively. The predicted secondary structures shown in FIGs. 8A and 8B are each in a non-active form in the absence of melamine.

Also, N1Mel06_I1_A0_D3C_CG (SEQ ID NO: 43) was produced by adding dG to the 3' end of N1Mel06_I1_A0_D3C_C shown in FIG. 8B. The predicted secondary structure thereof is shown in FIG. 8C. The predicted secondary structure shown in FIG. 8C is in an active form in the presence of melamine.

A nucleic acid sensor Mel06_dsG (SEQ ID NO: 40) having a melamine aptamer Mel06 (SEQ ID NO: 57, n = 12) as the binding nucleic acid molecule (A) and the following double-stranded DNAzyme (dsG) as the catalytic nucleic acid molecule (D) was produced. In the following sequence, the underlined part corresponds to dsG. The predicted secondary structure of this nucleic acid sensor is shown in FIG 8D. The predicted secondary structure shown in FIG 8D is in an active form in the presence of melamine.

dsG
5'-AGGGACGGGAAGAA-3' (SEQ ID NO: 59)
3'-TGGGAGGTATAAAA-5' (SEQ ID NO: 60)
Mel06_dsG (SEQ ID NO: 40) AGGGACGGGAAGAACGCTTTTTTTTTTTTGCGAAAATGTGGAGGGT

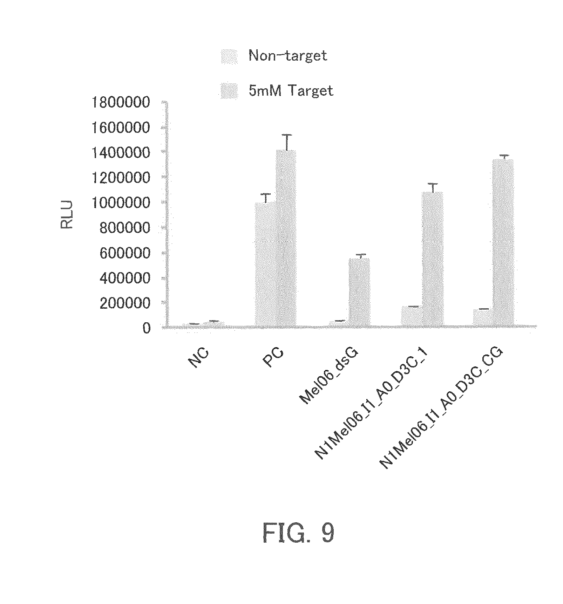

The relative chemiluminescence intensities (RLU) of the above nucleic acid sensors were measured in the same manner as in Example 3. The results thereof are shown in FIG. 9. FIG. 9 is a graph showing luminescence intensities (RLU) of the reaction solutions. Also, from the results shown in FIG. 9, the ratio (S/N) between the luminescence intensity in the presence of melamine and the luminescence intensity in the absence of melamine was determined for each sensor. The results thereof are shown in Table 9 below. As can be seen from FIG. 9 and Table 9, these sensors all could detect melamine. In particular, N1Mel06_I1_A0_D3C_CG, which is the sensor having dG added to the 5' end, exhibited a superior S/N.

**[Table 9]**

| Sensor No. | name | S/N |
|---|---|---|
| 1 | N1Mel06_I1_A0_D3_T00_C | 6.73 |
| 2 | Mel06_dsG | 9.78 |
| 3 | N1Mel06_I1_A0_D3C_1 | 6.53 |
| 5 | N1_Mel06_I1_A0_D3C_CG | 9.03 |
| PC | - | 1.51 |
| NC | - | 1.41 |

### (Example 6)

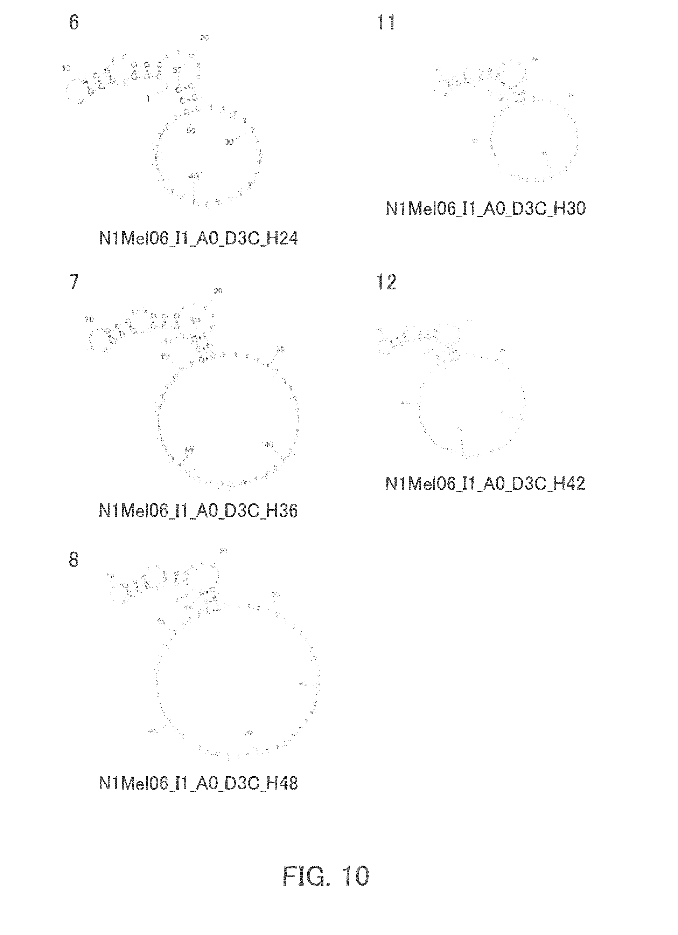

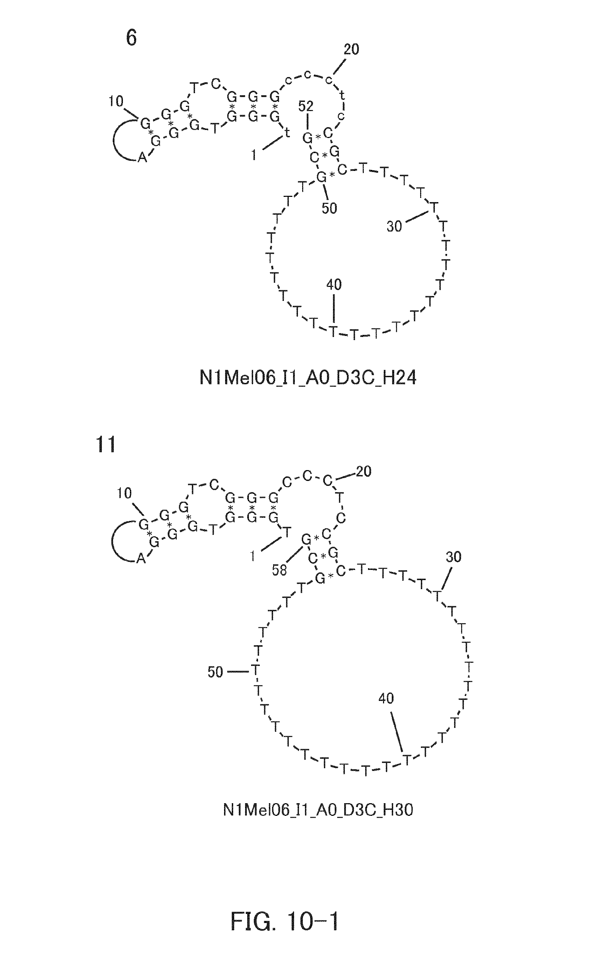

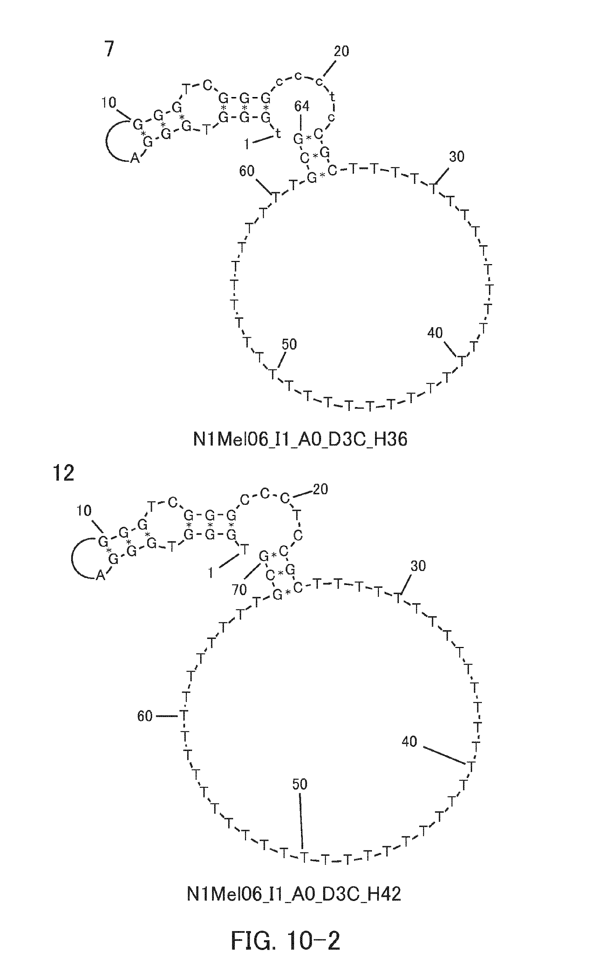

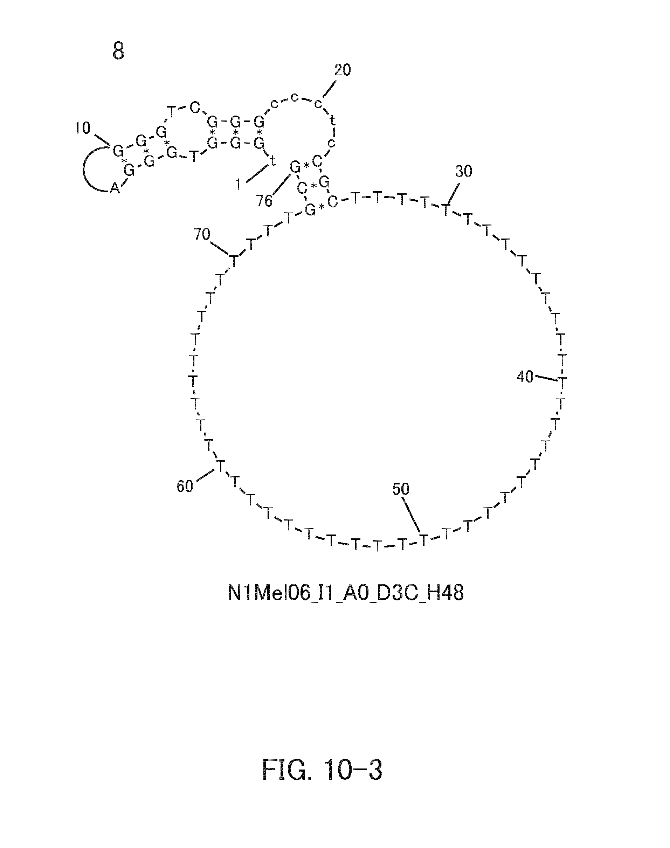

Modified sensors were produced by modifying the nucleic acid sensor N1Mel06_I1_A0_D3_T00_C (SEQ ID NO: 38) of Example 4. Specifically, in the active form predicted secondary structure of N1Mel06_I1_A0_D3_T00_C shown on the left in FIG. 6B, the 12-mer poly(dT) was modified to: 24-mer poly(dT) to produce N1Mel06_I1_A0_D3C_H24 (SEQ ID NO: 44); 30-mer to produce N1Mel06_I1_A0_D3C_H30 (SEQ ID NO: 52); 36-mer to produce N1Mel06_I1_A0_D3C_H36 (SEQ ID NO: 53); 42-mer to produce N1Mel06_I1_A0_D3C_H42 (SEQ ID NO: 54); and 48-mer to produce N1Mel06_I1_A0_D3C_H48 (SEQ ID NO: 46). The predicted secondary structures thereof are shown in FIG. 10. The predicted secondary structures shown in FIG. 10 are each in an active form in the presence of melamine.

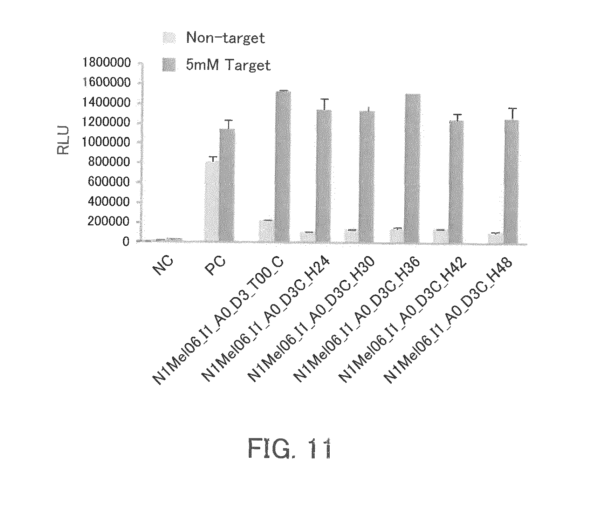

The relative chemiluminescence intensities (RLU) of the above nucleic acid sensors were measured in the same manner as in Example 4. The results thereof are shown in FIG. 11. FIG. 11 is a graph showing the luminescence intensities (RLU) of the reaction solutions. Also, from the results shown in FIG. 11, the ratio (S/N) between the luminescence intensity in the presence of melamine and the luminescence intensity in the absence of melamine was determined for each sensor. The results thereof are shown in Table 10 below. From these results, it was found that, by increasing the base length of the poly(dT) in the melamine aptamer to 20-mer or more, the sensitivity is further improved. Also, the measurement was carried out in the same manner using N1Mel06_I1_A0_D3_T00_C (SEQ ID NO: 38) with the final concentration of melamine being reduced to 300 µmol/L. As a result, the ratio S/N was 1.4. From this result, it was found that it is possible to detect melamine even when the concentration thereof is low.

**[Table 10]**

| Sensor No. | name | base length of poly(dT) | S/N |
|---|---|---|---|
| 1 | N1Mel06_I1_A0_D3_T00_C | 12 | 6.69 |
| 6 | N1Mel06_I1_A0_D3C_H24 | 24 | 11.83 |
| 11 | N1Mel06_I0_A0_D3C_H30 | 30 | 9.93 |
| 7 | N1Mel06_I1_A0_D3C_H36 | 36 | 11.00 |
| 12 | N1Mel06_I1_A0_D3C_H42 | 42 | 11.68 |
| 8 | N1Mel06_I1_A0_D3C_H48 | 48 | 10.70 |
| PC | - | - | 1.42 |
| NC | - | - | 1.41 |

### (Example 7)

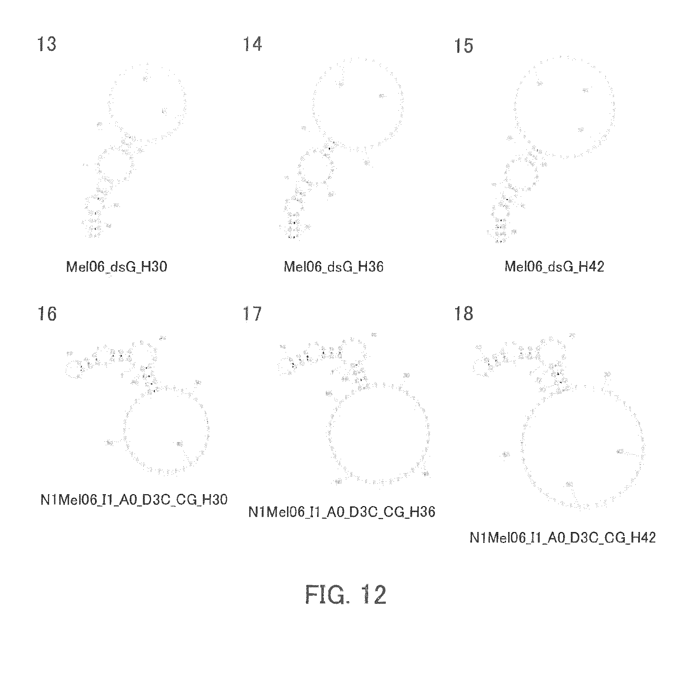

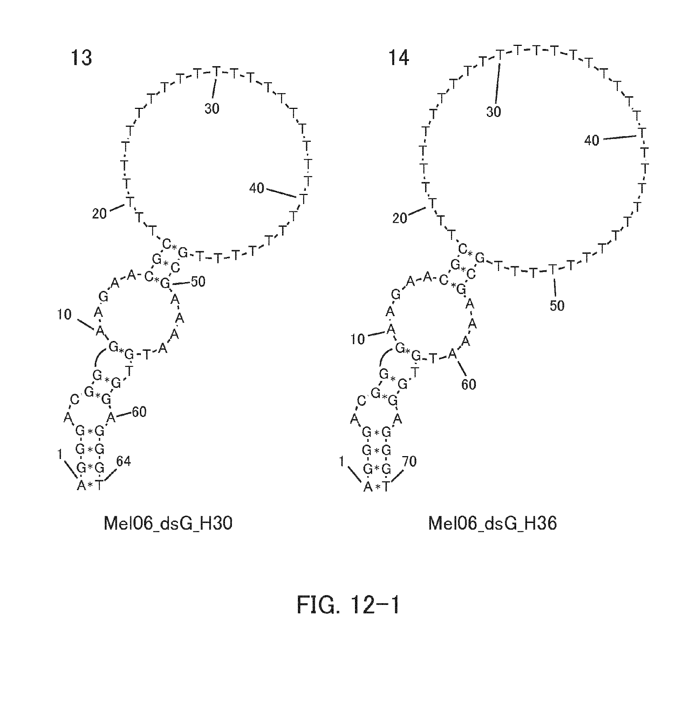

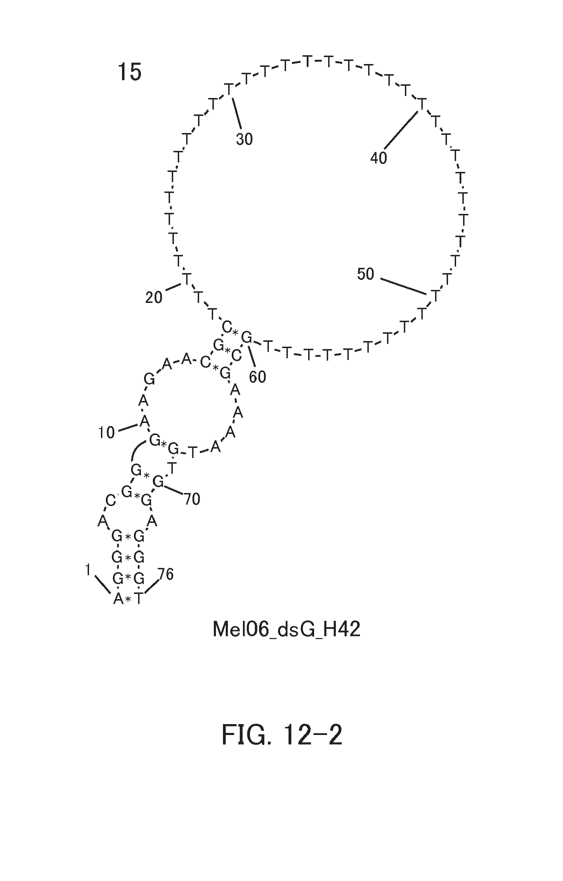

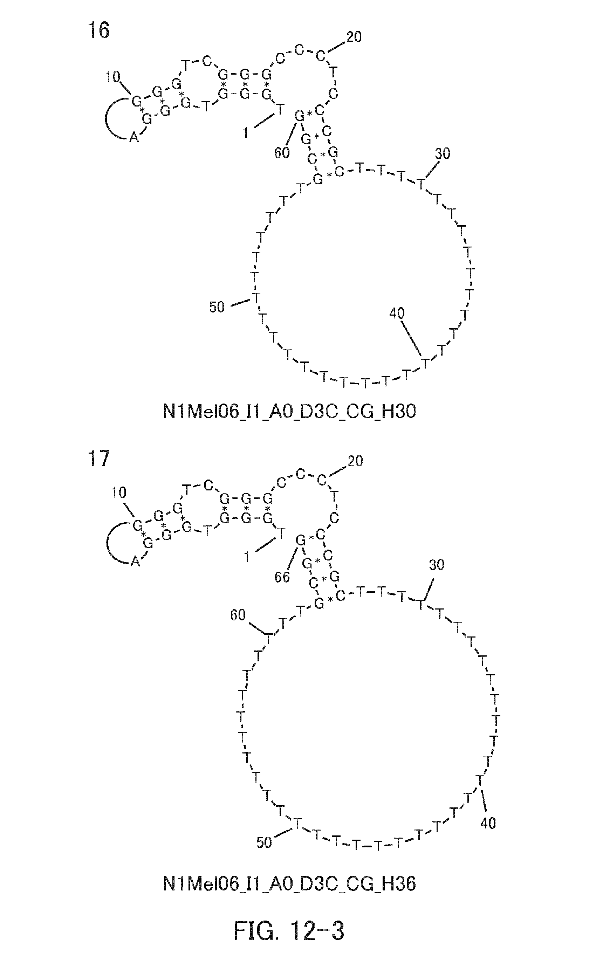

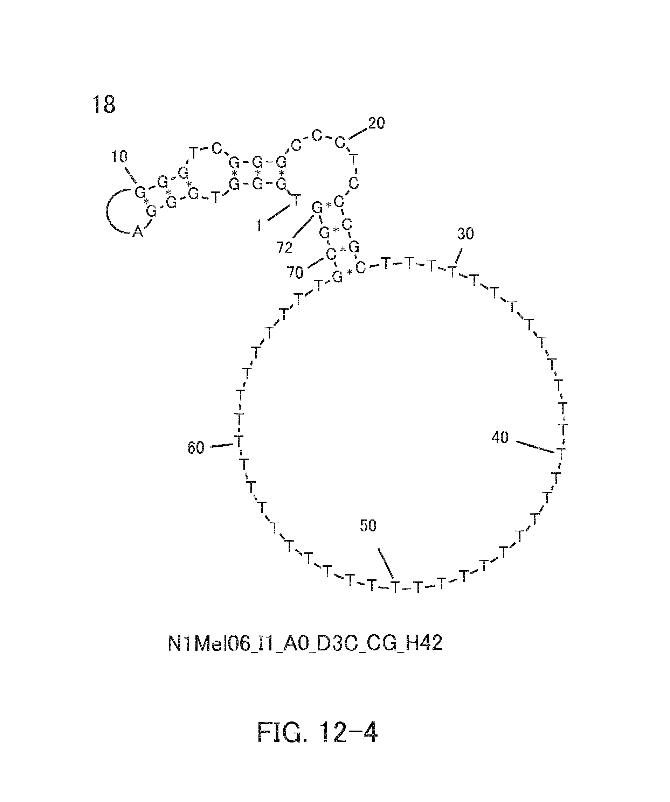

Modified sensors were produced by modifying the sensors Mel06_dsG (SEQ ID NO: 40) and N1Mel06_I1_A0_D3C_CG (SEQ ID NO: 43) of Example 5. Specifically, in the active form predicted secondary structures of Mel06_dsG and N1Mel06_I1_A0_D3C_CG shown in FIG. 11, the 12-mer poly(dT) was modified to 30-mer, 36-mer, or 42-mer poly(dT), whereby the following sensors were produced. The predicted secondary structures thereof are shown in FIG. 12. The predicted secondary structures shown in FIG. 12 are each in an active form in the presence of melamine.
Mel06_dsG_H30 (SEQ ID NO: 49)
Mel06_dsG_H36 (SEQ ID NO: 50)
Mel06_dsG_H42 (SEQ ID NO: 51)
N1Mel06_I1_A0_D3C_CG_H30 (SEQ ID NO: 52)
N1Mel06_I1_A0_D3C_CG_H36 (SEQ ID NO: 53)
N1Mel06_I1_A0_D3C_CG_H42 (SEQ ID NO: 54)

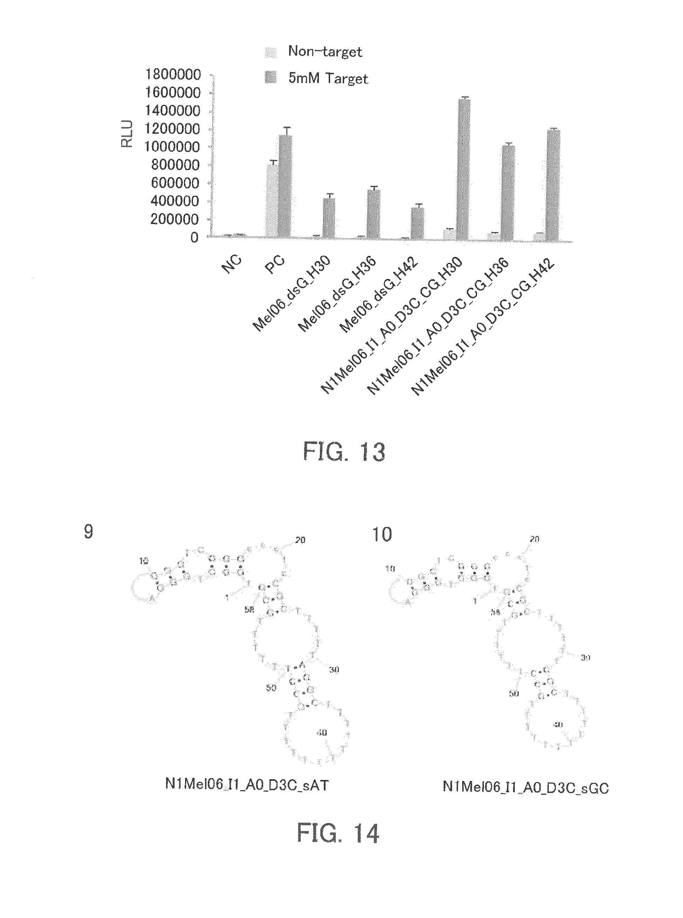

The relative chemiluminescence intensities (RLU) of the above nucleic acid sensors were measured in the same manner as in Example 3. The results thereof are shown in FIG. 13. FIG. 13 is a graph showing the luminescence intensities (RLU) of the reaction solutions. Also, from the results shown in FIG. 13, the ratio (S/N) between the luminescence intensity in the presence of melamine and the luminescence intensity in the absence of melamine was determined for each sensor. The results thereof are shown in Table 11 below. From these results, it was found that, by increasing the base length of the poly(dT) in the melamine aptamer to 30-mer or more, the sensitivity is further improved.

**[Table 11]**

| Sensor No. | name | base length of poly(dT) | S/N |
|---|---|---|---|
| 2 | Mel06_dsG | 12 | 9.78 |
| 13 | Mel06_dsG_H30 | 30 | 13.73 |
| 14 | Mel06_dsG_H36 | 36 | 18.25 |
| 15 | Mel06_dsG_H42 | 42 | 13.27 |
| 5 | N1Mel06_I1_A0_D3C_CG | 12 | 9.03 |
| 16 | N1Mel06_I1_A0_D3C_CG_H30 | 30 | 12.16 |
| 17 | N1Mel06_I1_A0_D3C_CG_H36 | 36 | 11.00 |
| 18 | N1Mel06_I1_A0_D3_CG_H42 | 42 | 11.68 |
| PC | - | - | 1.42 |
| NC | - | - | 1.41 |

### (Example 8)

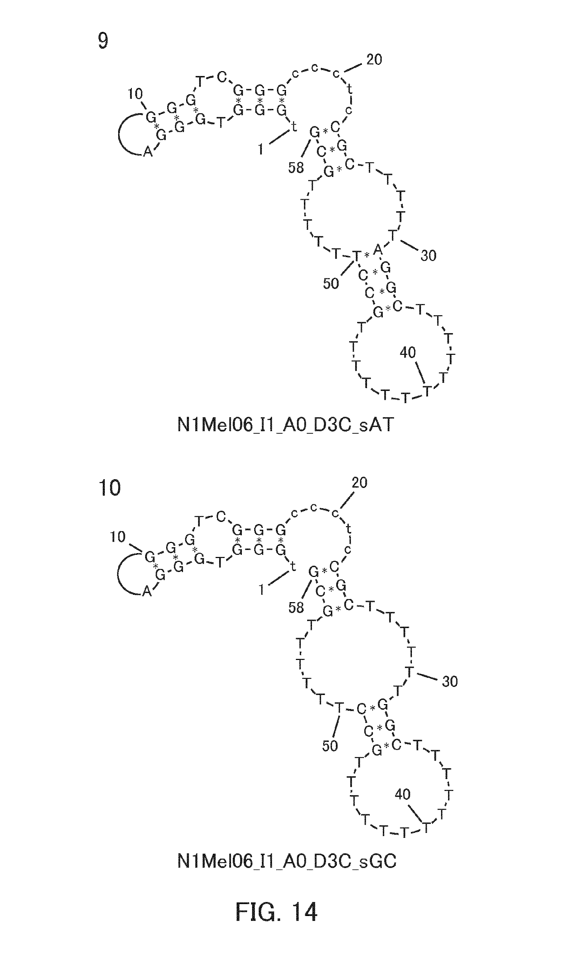

Modified sensors were produced by modifying the nucleic acid sensor N1Mel06_I1_A0_D3_T00_C of Example 4. Specifically, in the active form predicted secondary structure of N1Mel06_I1_A0_D3_T00_C shown on the left in FIG. 6B, the internal loop of poly(dT) was further added between the melanin aptamer and the DNAzyme. Thus, sensors N1Mel06_I1_A0_D3C_sAT (SEQ ID NO: 47) and N1Mel06_I1_A0_D3C_sGC (SEQ ID NO: 48) including the tandem type poly(dT)-containing melanin aptamer were produced. The predicted secondary structures thereof are shown in FIG. 14. The predicted secondary structures shown in FIG. 14 are each in an active form in the presence of melamine.

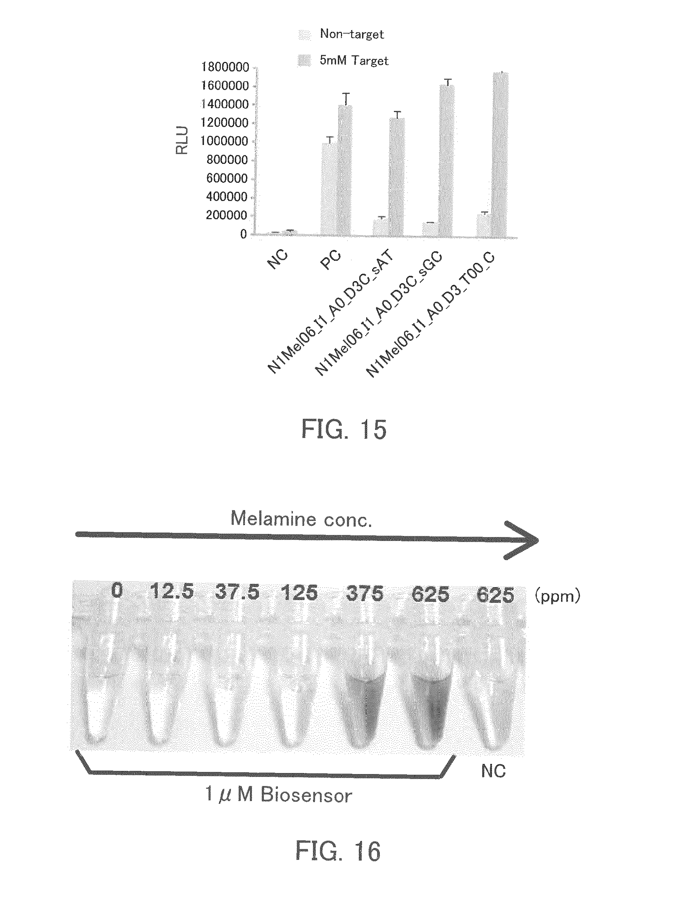

The relative chemiluminescence intensities (RLU) of the above nucleic acid sensors were measured in the same manner as in Example 3. The results thereof are shown in FIG. 15. FIG. 15 is a graph showing luminescence intensities (RLU) of the reaction solutions. Also, from the results shown in FIG. 15, the ratio (S/N) between the luminescence intensity in the presence of melamine and the luminescence intensity in the absence of melamine was determined for each sensor. The results thereof are shown in Table 12 below. These results demonstrate that it is possible to detect melanin also by providing a tandem type melamine aptamer in the sensor.

**[Table 12]**

| Sensor No. | name | S/N |
|---|---|---|
| 1 | N1Mel06_I1_A0_D3_T00_C | 6.73 |
| 9 | N1Mel06_I1_A0_D3C_sAT | 6.52 |
| 10 | N1Mel06_I1_A0_D3C_sGC | 10.39 |
| PC | - | 1.51 |
| NC | - | 1.41 |

### (Example 9)

Colorimetric analysis and chemiluminescence analysis of melamine were carried out using the nucleic acid sensor N1Mel06_I1_A0_D3_T00_C of Example 4.

### (1) Colorimetric analysis

To an Eppendorf tube, the following reagent 1, reagent 2, and reagent 3 were added in this order, and the mixture was reacted at 25°C for 5 minutes. Thereafter, the coloring of the reaction solution was examined through visual observation. Each concentration in the following compositions indicates the final concentration in the reaction solution. The final concentration of melamine in the reaction solution was set to 0, 12.5, 37.5, 125, 375, or 625 ppm (0,100 µmol/L, 300 µmol/L, 1 mmol/L, 3 mmol/L, or 5 mmol/L). As a substrate, ABTS was used.

| (Reagent 1) | |
|---|---|
| 1 µmol/L | nucleic acid sensor |
| 3 µmol/L | hemin |
| 50 mmol/L | Tris-HCl (pH 7.4) |
| 20 mmol/L | KCl |
| 0.05% (w/v) | Triton X-100 |

| (Reagent 2) | |
|---|---|
| predetermined concentration | melamine |

| (Reagent 3) | |
|---|---|
| 1 mmol/L | ABTS |
| 0.5 mmol/L | H₂O₂ |

As a negative control (NC), the visual observation was carried out in the same manner, except that the above reagent 1 without the nucleic acid sensor was used.

The results thereof are shown in FIG. 16. FIG. 16 shows the results regarding the color development of the reaction solutions. FIG. 16 is a photograph showing the coloring of the reaction solutions, in which the darker the color, the higher degree of coloring to blue is indicated. As can be seen from FIG. 16, in the reaction solutions with melamine concentrations of 375 ppm or higher, strong color development was observed. As described above, it is acceptable if the melamine concentration of about 500 ppm can be detected. Thus, it was found that, according to the nucleic acid sensor of the present example, it is possible to detect melamine by visual observation only.

### (2) Chemiluminescence analysis

The following reagent 1, reagent 2, and reagent 3 were added to a reaction vessel in this order. Then, with regard to 100 µL of the thus-obtained reaction solution, the change in relative chemiluminescence intensity (RLU) with time was measured at 25°C. The measurement was carried out using a luminometer (trade name: Lumitester PD-20, manufactured by Kikkoman Corporation). Each concentration in the following compositions indicates the final concentration in the reaction solution. The final concentration of melamine in the reaction solution was set to 0, 125, or 625 ppm (0, 1 mmol/L, or 5 mmol/L).

| (Reagent 1) | |
|---|---|
| 500 nmol/L | nucleic acid sensor |
| 250 nmol/L | hemin |
| 50 mmol/L | Tris-HCl (pH 7.4) |
| 20 mmol/L | KCl |
| 0.05% (w/v) | Triton X-100 |

| (Reagent 2) | |
|---|---|
| predetermined concentration | melamine |

| (Reagent 3) | |
|---|---|
| 25 µmol/L | L-012 |
| 25 µmol/L | H₂O₂ |

Table 13 below shows the luminescence intensities (RLU) of the reaction solution 30 seconds after the start of the reaction. As can be seen from Table 13, when the nucleic acid sensor of the present example was used, the luminescence intensity of the reaction solution with a melamine concentration of 125 ppm also was significantly different from that of the reaction solution with a melamine concentration of 0 ppm. This result demonstrates that, according to the nucleic acid sensor of the present example, it is possible to detect melamine even at a concentration of 125 ppm by measuring the luminescence intensity.

**[Table 13]**

| melamine concentration ppm | RLU |
|---|---|
| 0 | 145 |
| 125 | 176 |
| 625 | 1717 |

While the present invention has been described above with reference to embodiments, the present invention is by no means limited thereto. Various changes and modifications that may become apparent to those skilled in the art may be made in the configuration and specifics of the present invention without departing from the scope of the present invention.

This application claims priority from Japanese Patent Application No. 2012-167766 filed on July 27,2012. The entire disclosure of this Japanese patent application is incorporated herein by reference.

### Industrial Applicability

According to the nucleic acid sensor of the present invention, it is possible to switch ON-OFF of the catalytic function of the catalytic nucleic acid molecule (D) depending on whether or not the binding nucleic acid molecule (A) binds to melamine. Therefore, by detecting the catalytic function of the catalytic nucleic acid molecule (D), the presence or absence or the amount of melamine can be detected without difficulty. Furthermore, since the analysis device of the present invention uses the nucleic acid sensor as described above, for example, the reduction of the size of the device and the chipping of the device can be achieved and a simple analysis can be achieved even with respect to a number of specimens. Therefore, the present invention can be a very useful technique in the melamine analysis, for example.

### [Sequence Listing]

TF13021WO_ST25_2013.07.03.txt

## Claims

1. A nucleic acid sensor for melamine analysis comprising:
the following polynucleotide (x1), (x2), (x3), or (x4) that includes a catalytic nucleic acid molecule (D) that activates a catalytic function and a binding nucleic acid molecule (A) that binds to melamine:
(x1) a polynucleotide consisting of any one of base sequences of SEQ ID NOs: 1 to 14, wherein n and m are positive integers;
(x2) a polynucleotide consisting of a base sequence obtained by substitution, deletion, addition, and/or insertion of one or more bases in the base sequence of the polynucleotide (x1), wherein a catalytic function of the catalytic nucleic acid molecule (D) is inhibited in the absence of melamine and the catalytic function of the catalytic nucleic acid molecule (D) is activated in the presence of melamine;
(x3) a polynucleotide consisting of a base sequence having at least 80% identity to any base sequence of the polynucleotide (x1), wherein the catalytic function of the catalytic nucleic acid molecule (D) is inhibited in the absence of melamine and the catalytic function of the catalytic nucleic acid molecule (D) is activated in the presence of melamine; and
(x4) a polynucleotide consisting of a base sequence complementary to a polynucleotide that hybridizes to a polynucleotide consisting of any base sequence of the polynucleotide (x1) under a stringent condition, wherein the catalytic function of the catalytic nucleic acid molecule (D) is inhibited in the absence of melamine and the catalytic function of the catalytic nucleic acid molecule (D) is activated in the presence of melamine.

2. The nucleic acid sensor according to claim 1, wherein
in any one of the base sequences of SEQ ID NOs: 1 to 14, n of (T)n is 6 to 48 and m of (T)ₘ is 2 to 31.

3. The nucleic acid sensor according to claim 1 or 2, further comprising:
a linker sequence that links the catalytic nucleic acid molecule (D) and the binding nucleic acid molecule (A).

4. The nucleic acid sensor according to any one of claims 1 to 3, further comprising:
a 5' end-additional sequence linked to the 5' end of the polynucleotide (x1), (x2), (x3), or (x4).

5. The nucleic acid sensor according to claim 4, wherein
the 5' end-additional sequence has a length of 0 to 22 bases.

6. The nucleic acid sensor according to any one of claims 1 to 5, further comprising:
a 3' end-additional sequence linked to the 3' end of the polynucleotide (x1), (x2), (x3), or (x4).

7. The nucleic acid sensor according to claim 6, wherein
the 3' end-additional sequence has a length of 7 to 18 bases.

8. The nucleic acid sensor according to any one of claims 1 to 7, wherein the nucleic acid sensor comprises the following polynucleotide (X1), (X2), (X3), or (X4):
(X1) a polynucleotide consisting of any one of base sequences of SEQ ID NOs: 15 to 56;
(X2) a polynucleotide consisting of a base sequence obtained by substitution, deletion, addition, and/or insertion of one or more bases in the base sequence of the polynucleotide (X1), wherein the catalytic function of the catalytic nucleic acid molecule (D) is inhibited in the absence of melamine and the catalytic function of the catalytic nucleic acid molecule (D) is activated in the presence of melamine;
(X3) a polynucleotide consisting of a base sequence having at least 80% identity to any base sequence of the polynucleotide (X1), wherein the catalytic function of the catalytic nucleic acid molecule (D) is inhibited in the absence of melamine and the catalytic function of the catalytic nucleic acid molecule (D) is activated in the presence of melamine; and
(X4) a polynucleotide consisting of a base sequence complementary to a polynucleotide that hybridizes to a polynucleotide consisting of any base sequence of the polynucleotide (X1) under a stringent condition, wherein the catalytic function of the catalytic nucleic acid molecule (D) is inhibited in the absence of melamine and the catalytic function of the catalytic nucleic acid molecule (D) is activated in the presence of melamine.

9. A device for melamine analysis comprising:
a base material;
a nucleic acid sensor; and
a detection unit, wherein
the nucleic acid sensor and the detection unit are arranged on the base material,
the nucleic acid sensor is the nucleic acid sensor according to any one of claims 1 to 8, and
the detection unit is a detection unit detecting the catalytic function of the catalytic nucleic acid molecule (D) in the nucleic acid sensor.

10. The device according to claim 9, wherein
the nucleic acid sensor is linked to the base material via a linker.

11. The device according to claim 9 or 10, wherein
the nucleic acid sensor is arranged in the detection unit.

12. The device according to any one of claims 9 to 11, wherein
the detection unit detects a signal produced by the catalytic function of the catalytic nucleic acid molecule (D).

13. The device according to claim 12, wherein
the signal is an optical signal or an electrochemical signal.

14. The device according to any one of claims 9 to 13, further comprising:
a reagent unit, wherein
the reagent unit comprises a substrate for the catalytic function of the catalytic nucleic acid molecule (D).

15. A reagent for melamine analysis compsiring:
the nucleic acid sensor according to any one of claims 1 to 8.

16. The reagent according to claim 15, further comprising:
a substrate for the catalytic function of the catalytic nucleic acid molecule (D).

17. A method for melamine analysis comprising:
a contact step of bringing a sample into contact with the nucleic acid sensor for melamine analysis according to any one of claims 1 to 8; and
a detection step of detecting the catalytic function of the catalytic nucleic acid molecule (D) in the nucleic acid sensor to detect melamine in the sample.

18. The method according to claim 17, wherein the detection step is performed in the presence of a substrate for the catalytic function of the catalytic nucleic acid molecule (D).

19. A method for melamine analysis comprising:
a contact step of bringing a sample into contact with the device for analysis according to any one of claims 9 to 14; and
a detection step of detecting the catalytic function of the catalytic nucleic acid molecule (D) in the detection unit of the device to detect melamine in the sample.

20. The method according to claim 19, wherein the detection step is performed in the presence of a substrate for the catalytic function of the catalytic nucleic acid molecule (D).
